(19) Europäisches Patentamt
European Patent Office
Office européen des brevets

(11) **EP 2 367 840 B1**

(12) **EUROPEAN PATENT SPECIFICATION**

(45) Date of publication and mention
of the grant of the patent:
**12.08.2015 Bulletin 2015/33**

(51) Int Cl.:
***C07K 1/00*** *(2006.01)*    ***C07K 7/08*** *(2006.01)*
***C07K 14/00*** *(2006.01)*

(21) Application number: **09774880.0**

(22) Date of filing: **08.12.2009**

(86) International application number:
**PCT/EP2009/066640**

(87) International publication number:
**WO 2010/066740 (17.06.2010 Gazette 2010/24)**

(54) **SINGLE-CHAIN ANTIPARALLEL COILED COIL PROTEINS**

ANTIPARALLELE COILED-COIL-EINZELKETTENPROTEINE

PROTÉINES SURENROULÉES ANTIPARALLÈLES À CHAÎNE UNIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **08.12.2008 US 120642 P**

(43) Date of publication of application:
**28.09.2011 Bulletin 2011/39**

(73) Proprietor: **Complix N.V.**
**3590 Diepenbeek (BE)**

(72) Inventors:
• **DESMET, Johan**
**B-8500 Kortrijk (BE)**
• **LASTERS, Ignace**
**B-2018 Antwerpen (BE)**
• **LOVERIX, Stefan**
**B-1740 Ternat (BE)**

(74) Representative: **Hoffmann Eitle**
**Patent- und Rechtsanwälte PartmbB**
**Arabellastraße 30**
**81925 München (DE)**

(56) References cited:
**EP-A1- 2 161 278         WO-A2-2005/077103**
**WO-A2-2009/030780**

• **PASCUAL J ET AL: "Solution structure of the spectrin repeat: a left-handed antiparallel triple-helical coiled-coil" JOURNAL OF MOLECULAR BIOLOGY, LONDON, GB, vol. 273, no. 3, 31 October 1997 (1997-10-31), pages 740-751, XP004453905 ISSN: 0022-2836**
• **WOOLFSON D N: "THE DESIGN OF COILED-COIL STRUCTURES AND ASSEMBLIES" ADVANCES IN PROTEIN CHEMISTRY, ACADEMIC PRESS, NEW YORK, NY, US, vol. 70, 1 January 2005 (2005-01-01), pages 79-112, XP008073019 ISSN: 0065-3233**
• **PARRY DAVID A D ET AL: "Fifty years of coiled-coils and alpha-helical bundles: a close relationship between sequence and structure." JOURNAL OF STRUCTURAL BIOLOGY SEP 2008, vol. 163, no. 3, September 2008 (2008-09), pages 258-269, XP002575056 ISSN: 1095-8657**
• **KIYOKAWA TOMOHIRO ET AL: "Engineering of the hydrophobic core of an alpha-helical coiled coil" BIOPOLYMERS, NEW YORK, NY, US, vol. 55, no. 5, 1 September 2000 (2000-09-01), pages 407-414, XP008105332 ISSN: 0006-3525 [retrieved on 2001-02-15]**
• **OAKLEY M G ET AL: "The design of antiparallel coiled coils" CURRENT OPINION IN STRUCTURAL BIOLOGY, ELSEVIER LTD, GB, vol. 11, no. 4, 1 August 2001 (2001-08-01), pages 450-457, XP002551052 ISSN: 0959-440X**
• **EFIMOV V P ET AL: "Fibritin Encoded by Bacteriophage T4 Gene wac has a Parallel Triple-stranded .alpha.-Helical Coiled-coil Structure" JOURNAL OF MOLECULAR BIOLOGY, LONDON, GB, vol. 242, 1 January 1994 (1994-01-01), pages 470-486, XP002523719 ISSN: 0022-2836**

**(Cont. next page)**

Remarks:
   The file contains technical information submitted after the application was filed and not included in this specification

Remarks:
   The file contains technical information submitted after the application was filed and not included in this specification

SEQUENCE LISTING

<110> Complix NV

<120> Single-chain antiparallel coiled coil proteins

<130> 138956

<160> 23

<170> PatentIn version 3.4

<210> 1
<211> 24
<212> PRT
<213> Artificial

<220>
<223> Synthetic peptide

<400> 1

```
Ala Ile Ala Ala Ile Gln Lys Gln Ile Ala Ala Ile Gln Lys Gln Ile
1               5                   10                  15

Ala Ala Ile Gln Lys Gln Ile Ala
            20
```

<210> 2
<211> 29
<212> PRT
<213> Artificial

<220>
<223> Synthetic peptide

<220>
<221> MISC_FEATURE
<222> (1)..(1)
<223> Acetylated N-terminus

<220>
<221> MISC_FEATURE
<222> (29)..(29)
<223> Amidated C-terminus

<400> 2

```
Met Ser Ile Glu Glu Ile Gln Lys Gln Gln Ala Ala Ile Gln Lys Gln
1               5                   10                  15

Ile Ala Ala Ile Gln Lys Gln Ile Tyr Arg Met Thr Pro
            20                  25
```

<210> 3
<211> 29
<212> PRT
<213> Artificial

<220>
<221> MISC_FEATURE
<222> (1)..(1)
<223> Acetylated N-terminus

<220>
<221> MISC_FEATURE
<222> (29)..(29)
<223> Amidated C-terminus

<400> 3

```
Met Ser Ile Glu Glu Ile Gln Lys Gln Ile Ala Ala Ile Gln Lys Gln
1               5                   10                  15

Ile Ala Ala Ile Gln Lys Gln Ile Tyr Arg Met Thr Pro
                20                  25
```

<210> 4
<211> 122
<212> PRT
<213> Artificial

<220>
<223> Synthetic peptide (scQ2al_L8)

<400> 4

```
Met Gly His His His His His His His His His Ser Ser Gly His
1               5                   10                  15

Ile Glu Gly Arg His Met Ser Ile Glu Glu Ile Gln Lys Gln Ile Ala
            20                  25                  30

Ala Ile Gln Lys Gln Ile Ala Ala Ile Gln Lys Gln Ile Tyr Arg Met
            ·35                 40                  45

Thr Gly Gly Ser Gly Gly Gly Ser Gly Met Ser Ile Glu Glu Ile Gln
        50                  55                  60

Lys Gln Ile Ala Ala Ile Gln Lys Gln Ile Ala Ala Ile Gln Lys Gln
65                  70                  75                  80

Ile Tyr Arg Met Thr Gly Gly Ser Gly Gly Gly Ser Gly Met Ser Ile
                85                  90                  95

Glu Glu Ile Gln Lys Gln Ile Ala Ala Ile Gln Lys Gln Ile Ala Ala
            100                 105                 110

Ile Gln Lys Gln Ile Tyr Arg Met Thr Pro
        115                 120
```

<211> 138
<212> PRT
<213> Artificial

<220>
<223> Synthetic peptide (scQ2al_L16)

<400> 5

24

```
Met Gly His His His His His His His His His Ser Ser Gly His
1             5                  10                  15

Ile Glu Gly Arg His Met Ser Ile Glu Glu Ile Gln Lys Gln Ile Ala
            20              25                  30

Ala Ile Gln Lys Gln Ile Ala Ala Ile Gln Lys Gln Ile Tyr Arg Met
        35              40                  45

Thr Gly Gly Ser Gly Gly Gly Ser Gly Gly Gly Ser Gly Gly Gly Ser
    50              55                  60

Gly Met Ser Ile Glu Glu Ile Gln Lys Gln Ile Ala Ala Ile Gln Lys
65              70                  75                  80

Gln Ile Ala Ala Ile Gln Lys Gln Ile Tyr Arg Met Thr Gly Gly Ser
            85                  90                  95

Gly Gly Gly Ser Gly Gly Gly Ser Gly Gly Gly Ser Gly Met Ser Ile
            100                 105                 110

Glu Glu Ile Gln Lys Gln Ile Ala Ala Ile Gln Lys Gln Ile Ala Ala
            115                 120                 125

Ile Gln Lys Gln Ile Tyr Arg Met Thr Pro
            130                 135
```

<210> 6
<211> 97
<212> PRT
<213> Artificial

<220>
<223> Synthetic peptide (short_L6)

<400> 6

```
Met Gly His His His His His His His His His Ser Ser Gly His
1             5                  10                  15

Ile Glu Gly Arg His Met Ser Ile Glu Glu Ile Gln Lys Gln Ile Ala
            20              25                  30

Ala Ile Gln Lys Gln Ile Tyr Arg Met Thr Gly Gly Ser Gly Gly Gly
```

```
Met Ser Ile Glu Glu Ile Gln Lys Gln Ile Ala Ala Ile Gln Lys Gln
    50                  55                  60

Ile Tyr Arg Met Thr Gly Gly Ser Gly Gly Gly Met Ser Ile Glu Glu
65                  70                  75                  80

Ile Gln Lys Gln Ile Ala Ala Ile Gln Lys Gln Ile Tyr Arg Met Thr
                85                  90                  95

Pro
```

<210> 7
<211> 105
<212> PRT
<213> Artificial

<220>
<223> Synthetic peptide (short_L10)

<400> 7

```
Met Gly His His His His His His His His His His Ser Ser Gly His
1                   5                   10                  15

Ile Glu Gly Arg His Met Ser Ile Glu Glu Ile Gln Lys Gln Ile Ala
            20                  25                  30

Ala Ile Gln Lys Gln Ile Tyr Arg Met Thr Gly Gly Ser Gly Gly Gly
            35                  40                  45

Ser Gly Gly Gly Met Ser Ile Glu Glu Ile Gln Lys Gln Ile Ala Ala
    50                  55                  60

Ile Gln Lys Gln Ile Tyr Arg Met Thr Gly Gly Ser Gly Gly Gly Ser
65                  70                  75                  80

Gly Gly Gly Met Ser Ile Glu Glu Ile Gln Lys Gln Ile Ala Ala Ile
                85                  90                  95

Gln Lys Gln Ile Tyr Arg Met Thr Pro
                100                 105
```

<210> 8
<211> 113
<212> PRT
<213> Artificial

<220>
<223> Synthetic peptide (short_L14)

<400> 8

```
Met Gly His His His His His His His His His Ser Ser Gly His
1               5                   10                  15

Ile Glu Gly Arg His Met Ser Ile Glu Glu Ile Gln Lys Gln Ile Ala
            20                  25                  30

Ala Ile Gln Lys Gln Ile Tyr Arg Met Thr Gly Gly Ser Gly Gly Gly
        35                  40                  45

Ser Gly Gly Gly Ser Gly Gly Gly Met Ser Ile Glu Glu Ile Gln Lys
    50                  55                  60

Gln Ile Ala Ala Ile Gln Lys Gln Ile Tyr Arg Met Thr Gly Gly Ser
65                  70                  75                  80

Gly Gly Gly Ser Gly Gly Gly Ser Gly Gly Gly Met Ser Ile Glu Glu
                85                  90                  95

Ile Gln Lys Gln Ile Ala Ala Ile Gln Lys Gln Ile Tyr Arg Met Thr
        100                 105                 110

Pro
```

<210> 9
<211> 121
<212> PRT
<213> Artificial

<220>
<223> Synthetic peptide (short_L18)

<400> 9

```
Met Gly His His His His His His His His His His Ser Ser Gly His
1                   5                   10                  15

Ile Glu Gly Arg His Met Ser Ile Glu Glu Ile Gln Lys Gln Ile Ala
                20                  25                  30

Ala Ile Gln Lys Gln Ile Tyr Arg Met Thr Gly Gly Ser Gly Gly Gly
                35                  40                  45

Ser Gly Gly Gly Ser Gly Gly Gly Ser Gly Gly Gly Met Ser Ile Glu
        50                  55                  60

Glu Ile Gln Lys Gln Ile Ala Ala Ile Gln Lys Gln Ile Tyr Arg Met
65                  70                  75                  80

Thr Gly Gly Ser Gly Gly Gly Ser Gly Gly Gly Ser Gly Gly Gly Ser
                85                  90                      95

Gly Gly Gly Met Ser Ile Glu Glu Ile Gln Lys Gln Ile Ala Ala Ile
            100                 105                 110

Gln Lys Gln Ile Tyr Arg Met Thr Pro
            115             120
```

<210> 10
<211> 138
<212> PRT
<213> Artificial

<220>
<223> Synthetic peptide (scQ2al_L16 derivative)

<400> 10

```
Met Gly His His His His His His His His His Ser Ser Gly His
1             5                 10                15

Ile Glu Gly Arg His Met Ser Ile Glu Glu Ile Gln Lys Gln Ile Ala
        20                  25                  30

Ala Ile Gln Lys Gln Ile Ala Ala Ile Gln Lys Gln Ile Tyr Arg Met
        35                  40                  45

Thr Gly Gly Ser Gly Gly Gly Ser Gly Gly Gly Ser Gly Gly Gly Ser
    50                  55                  60

Gly Trp Ser Ile Glu Glu Ile Gln Lys Gln Ile Ala Ala Ile Gln Lys
65                  70                  75                  80

Gln Ile Ala Ala Ile Gln Lys Gln Ile Tyr Arg Met Thr Gly Gly Ser
            85                  90                  95

Gly Gly Gly Ser Gly Gly Gly Ser Gly Gly Gly Ser Gly Met Ser Ile
            100                 105                 110

Glu Glu Ile Gln Lys Gln Ile Ala Ala Ile Gln Lys Gln Ile Ala Ala
            115                 120                 125

Ile Gln Cys Gln Ile Tyr Arg Met Thr Pro
        130                 135
```

<210> 11
<211> 7
<212> PRT
<213> Artificial

<220>
<223> Heptad repeat sequence

<400> 11

```
Ile Ala Ala Ile Gln Lys Gln
1               5
```

<210> 12
<211> 6
<212> PRT
<213> Artificial

<220>
<223> Flanking sequence

<220>
<221> MISC_FEATURE
<222> (6)..(6)
<223> Amidated C-terminus

<400> 12

```
                              Ile Tyr Arg Met Thr Pro
                              1               5
```

<210> 13
<211> 23
<212> PRT
<213> Artificial

<220>
<223> Flanking sequence

<400> 13

```
        Met Gly His His His His His His His His His His Ser Ser Gly His
        1               5                   10                  15

        Ile Glu Gly Arg His Met Ser
                        20
```

<210> 14
<211> 10
<212> PRT
<213> Artificial

<220>
<223> 10-His tag

<400> 14

```
                              His His His His His His His His His His
                              1               5                   10
```

<210> 15
<211> 5
<212> PRT
<213> Artificial

<220>
<223> Factor Xa cleavage site

<400> 15

```
                              Ile Glu Gly Arg His
                              1               5
```

<210> 16

<211> 25
<212> PRT
<213> Artificial

<220>
<223> Heptad repeat sequence

<400> 16

```
Ile Glu Glu Ile Gln Lys Gln Ile Ala Ala Ile Gln Lys Gln Ile Ala
1               5                   10                  15

Ala Ile Gln Lys Gln Ile Tyr Arg Met
            20              25
```

<210> 17
<211> 18
<212> PRT
<213> Artificial

<220>
<223> Heptad repeat sequence

<400> 17

```
Ile Glu Glu Ile Gln Lys Gln Ile Ala Ala Ile Gln Lys Gln Ile Tyr
1               5                   10                  15

Arg Met
```

<210> 18
<211> 11
<212> PRT
<213> Artificial

<220>
<223> Gly/Ser linker with flanking residues

<400> 18

```
Thr Gly Gly Ser Gly Gly Gly Ser Gly Met Ser
1               5                   10
```

<210> 19
<211> 19
<212> PRT
<213> Artificial

<220>
<223> Gly/Ser linker with flanking residues

<400> 19

```
        Thr Gly Gly Ser Gly Gly Gly Ser Gly Gly Gly Ser Gly Gly Gly Ser
        1               5                   10                  15
```

```
        Gly Met Ser
```

<210> 20
<211> 6
<212> PRT
<213> Artificial

<220>
<223> Gly/Ser linker

<400> 20

```
                            Gly Gly Ser Gly Gly Gly
                            1               5
```

<210> 21
<211> 10
<212> PRT
<213> Artificial

<220>
<223> Gly/Ser linker

<400> 21

```
                    Gly Gly Ser Gly Gly Gly Ser Gly Gly Gly
                    1               5                   10
```

<210> 22
<211> 14
<212> PRT
<213> Artificial

<220>
<223> Gly/Ser linker

<400> 22

```
            Gly Gly Ser Gly Gly Gly Ser Gly Gly Gly Ser Gly Gly Gly
            1               5                   10
```

<210> 23
<211> 18
<212> PRT
<213> Artificial

<220>
<223> Gly/Ser linker

<400> 23

```
Gly Gly Ser Gly Gly Gly Ser Gly Gly Gly Ser Gly Gly Gly Ser Gly
     1               5                   10                  15


Gly Gly
```

**Description**

FIELD OF THE INVENTION

The present invention is in the field of molecular biology and relates to thermodynamically stable, single-chain proteins that essentially consist of a triple-stranded, antiparallel, alpha-helical coiled coil scaffold structure in aqueous solutions. Such molecules are very stable and tolerant to amino acid substitutions. Accordingly, they meet the basic requirements of a protein-based scaffold. This scaffold exhibiting therapeutic, diagnostic and/or purification capacities, is usable in the field of drug discovery, analytical research, purification technology and as a model for improving the design of new proteinaceous (protein-like) scaffold structures. Protein-based scaffold molecules are often considered as the 'next-generation' class of compounds for molecular recognition, which increasingly compete with immunoglobulin-based compounds. Accordingly, the compounds of the present invention offer an alternative approach to immunoglobulins, and an additional type of protein-based (proteinaceous) scaffold.

BACKGROUND OF THE INVENTION

Triple-stranded (3-stranded) alpha-helical coiled coil complexes (coiled coil structures, coiled coils) are formed in solution by the association (coming together) of individual (separate, monomeric, free) peptide molecules into trimers (3-molecule complexes). The individual peptides typically comprise one or more heptad repeats (heptad units, heptads) which provide the thermodynamic driving force for such association.
An important practical problem encountered with the formation of trimeric complexes is the fact that such reactions are extremely dependent on the concentration. Therefore, unless the thermodynamic driving force is extremely strong (i.e., only if the heptads form extremely tight interactions), one has to apply relatively high concentrations in order for the trimeric complex to form. High concentrations can have multiple adverse effects when applied to (administered as) pharmaceutical compounds. In contrast to trimeric complexes, the formation of (folding of) single-chain coiled coil structures of the present invention is not dependent on their concentration in solution. The present invention therefore intends to provide a solution to the problem of concentration dependence.
A second problem related to the usage of peptidic oligomeric (multimeric) complexes is that the constituting peptides are difficult to produce (synthesize) via recombinant methods (i.e., using molecular biological techniques). This contrasts with stably folded single-chain proteins, which are ideally suited for recombinant synthesis. Thus, the present invention provides a solution to technical problems relating to synthesis of trimeric coiled coil scaffolds in peptidic form.
Thirdly, the present invention aims at providing a practical solution to the problem of creating heterotrimeric coiled coil structures. The oligomeric nature of peptidic coiled coils is in general defined by the number of associated peptides (e.g., 2, 3, 4, for dimeric, trimeric, tetrameric complexes, respectively), their mutual orientation (e.g., parallel or antiparallel) and their chemical similarity (i.e., their amino acid sequence with optional derivatization; e.g., homotrimeric coiled coils are formed by three identical peptides, heterotrimeric coiled coils comprise at least one different-sequence or derivatized peptide). Oligomeric coiled coils can be obtained in aqueous solution by mixing non-identical peptides. Then, after a sufficiently long incubation time, a distribution of homo- and heteromeric coiled coils will form, depending primarily on the latter's thermodynamic fitness (stability, free energy, quality of association). In view of the complicated atomic interactions that lie at the basis of thermodynamic fitness and, thereby, oligomeric preferences (distributions), the creation of specific, desired types of heteromeric coiled coils is technically hard to control. It is in this respect that the present invention provides a practical solution to a technical problem: since the coiled coil-forming peptide fragments are covalently linked together into a single chain (through suitably chosen linker fragments), their propensity to form coiled coil structures of predefined (desired) nature is considerably enhanced compared to equivalent coiled coils consisting of assemblies of free peptides. Consequently, the construction of specific heteromeric (e.g., heterotrimeric) coiled coils is considerably facilitated. In addition, the single-chain coiled coil format also offers the advantage of avoiding (or considerably reducing the risk of) formation of undesired (e.g., non-functional) types of association. In general, the single-chain format, which applies to all embodiments of the present invention, provides a practical solution to controlling and preserving the fold specificity of a trimeric coiled coil wherein the coiled coil-forming peptide fragments are (optionally) different in amino acid sequence.
All embodiments of the present invention relate to 'single-chain' yet 'triple-stranded' alpha-helical coiled coil structures. For the sake of clarity, it is explained here (and discussed further below in detail) that the property 'single-chain' relates to the complete molecules of the present invention, whereas the property 'triple-stranded' relates to the alpha-helical coiled coil part within these molecules. Wherever the description 'single-chain coiled coil' is used, this should be interpreted as a tight association between (three) coiled coil-forming peptide fragments that are covalently interconnected by (two) structurally flexible linker fragments; the said peptide and linker fragments together form one protein molecule consisting of a single, contiguous, amino acid chain. The single-chain coiled coil proteins of the present invention are also monomers (monomeric protein molecules in solution), which is not to be confused with the trimeric nature of the coiled coil structure that is contained within each such protein. The isolated single-chain protein is defined in the claims.

The vast majority of triple-stranded coiled coil structures in the Protein Data Bank (hereinafter referred to as PBD) are parallel coiled coils, i.e. of the type 'parallel alpha-helical peptides'. This means that the coiled coils exist as complexes (non-covalent associations) of three alpha-helical peptides per structure and wherein the helices are oriented in a parallel configuration (orientation). Very rarely, one of the three alpha-helices is oriented antiparallel to the other two (which are then parallel to each other). Such antiparallel arrangement is exceptional in natural proteins and has never been observed in the form of a regular coiled coil structure that is composed of, and stabilized by, conventional heptad repeat motifs. TABLE 1 shows an exhaustive list of 179 peptidic triple-stranded coiled coil complexes from the PDB, 175 of which are parallel and only 4 are antiparallel. This suggests that a parallel orientation is the most stable configuration for peptidic trimeric coiled coils. A likely reason for the abundance of parallel configurations is the preservation of 3-fold symmetry, which allows a maximal number of optimal contacts. In contrast, all embodiments of the present invention relate to single-chain coiled coils which adopt an antiparallel orientation. In view of the rare examples of antiparallel triple-stranded coiled coil structures in the PDB, the design and creation of such structures is absolutely not obvious. For example, such work is not only complicated by the lack of representative template (example) structures, it is also a priori unclear whether antiparallel coiled coils can be developed with core interactions of comparable quality as observed in parallel triple-stranded coiled coils. In view of the previous, one of the major inventive aspects of the present invention is the unanticipated finding that highly stable antiparallel triple-stranded coiled coils can be obtained. This indicates that core residues at conventional heptad repeat positions can also make quasi-optimal interactions in an antiparallel configuration, which was previously unknown.

## SUMMARY OF THE INVENTION

The inventors have constructed single-chain triple-stranded coiled coil protein structures that were anticipated to fold in parallel configuration, but with linker fragments that were significantly too short to permit this type of folding. Unexpectedly, it was found that the latter constructs had the same physical properties (alpha-helical content, thermal stability, solubility, etc) as variants with very long linkers. While, in general, constructs with physically too short linkers provoke unfolding of the structure, the trimeric scaffold structures of the present invention unexpectedly exhibited high thermal stability under conditions significantly deviating from physiological conditions, e.g. in 8 M urea, or at temperatures exceeding 90°C, and this irrespective of the linker lengths. These findings strongly suggest that the molecules of the present invention fold into an antiparallel configuration. The latter was also confirmed by NMR spectroscopy. Such novel coiled coil structures consequently are of high value for many scaffold-based applications.

The present invention relates to a class of isolated novel single-chain proteins of the formula HRS1-L1-HRS2-L2-HRS3, wherein HRS1, L1, HRS2, L2 and HRS3 represent amino acid sequence fragments that are covalently interconnected, <page 6a> said protein spontaneously folding in aqueous solution by way of the HRS1, HRS2 and HRS3 fragments forming a triple-stranded, antiparallel, alpha-helical coiled coil structure, and wherein

each of HRS1, HRS2 and HRS3 is independently a heptad repeat sequence that is characterized by a n-times repeated 7-residue pattern of amino acid types, represented as (a-b-c-d-e-f-g-)n or (d-e-f-g-a-b-c-)n and a C-terminal partial heptad repeat ending with a heptad core residue located at an a- or d-position, wherein the pattern elements 'a' to 'g' denote conventional heptad positions at which said amino acid types are located and n is a number equal to or greater than 2, and at least 50%, 70%, 90%, or 100% of the conventional heptad positions 'a' and 'd' are occupied by amino acids selected from the group consisting of valine, isoleucine, leucine, methionine, phenylalanine, tyrosine, tryptophan, histidine, glutamine, threonine, serine, alanine derivatives thereof, wherein at least 50%, 70%, 90%, or wherein 100% of the conventional heptad positions 'b', 'c', 'e', 'f' and 'g' are occupied by amino acids selected from the group consisting of glycine, alanine, cysteine, serine, threonine, histidine, asparagine, aspartic acid, glutamine, glutamic acid, lysine, arginine or derivatives thereof, the resulting distribution of amino acid types enabling the identification of said heptad repeat sequences, and

each of L1 and L2 is independently a linker consisting of 1 to 30 amino acid residues, this linker including any amino acid residue that cannot be unambiguously assigned to a heptad repeat sequence, and

L1 and L2 have an amino acid composition comprising at least 50% amino acids selected from the group consisting of glycine, alanine, serine, threonine, proline or derivatives thereof.

## BRIEF DESCRIPTION OF THE DRAWINGS

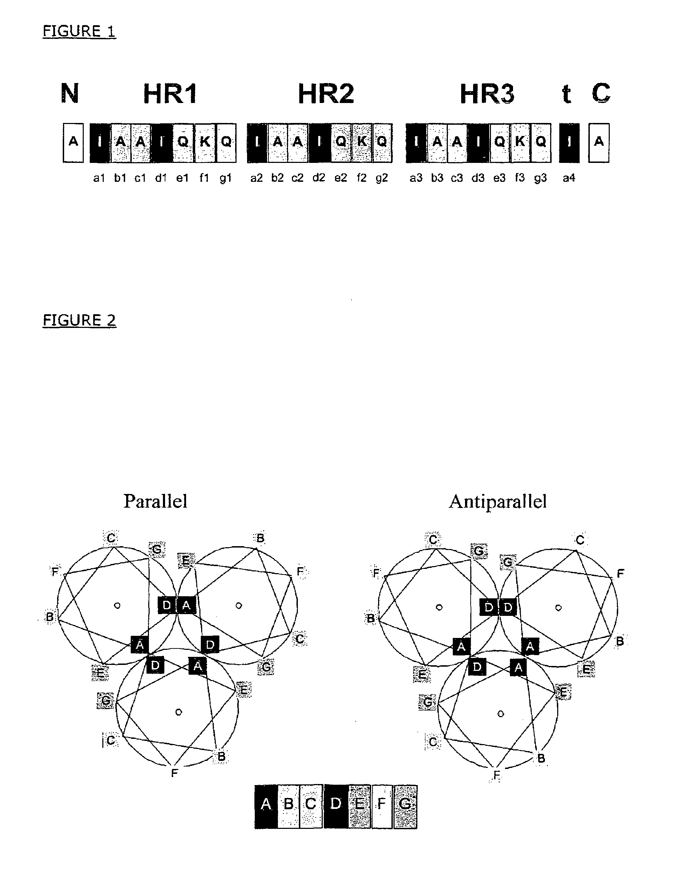

Figure 1 illustrates an amino acid sequence of a synthetic peptide comprising heptad repeats. The amino acid sequence is presented in single-letter notation, wherein A refers to alanine, I to isoleucine, Q to glutamine, and K to lysine. The peptide comprises heptad repeats (HRx), core residues (black boxes), non-core residues (gray boxes) and flanking regions (white boxes). The peptide further comprises a C-terminal heptad core residue labeled 't'. The peptide further comprises N- and C-terminal flanking fragments labeled 'N' and 'C', respectively. Each heptad repeat residue is further annotated with indices 'a' to 'g' and a number corresponding to the heptad repeat number. Core residues are located at a- and d-positions.

Figure 2 illustrates the principles of a triple-stranded, alpha-helical coiled coil complex. The figure provides a helical wheel representation of triple-stranded, alpha-helical coiled coil structures. The left panel shows a top view on a parallel coiled coil. The right panel shows a top view on an antiparallel coiled coil. The middle panel shows the linear sequence of heptad repeat positions. Only one heptad repeat is displayed for clarity reasons. Different shades are used to indicate specific topological positions.

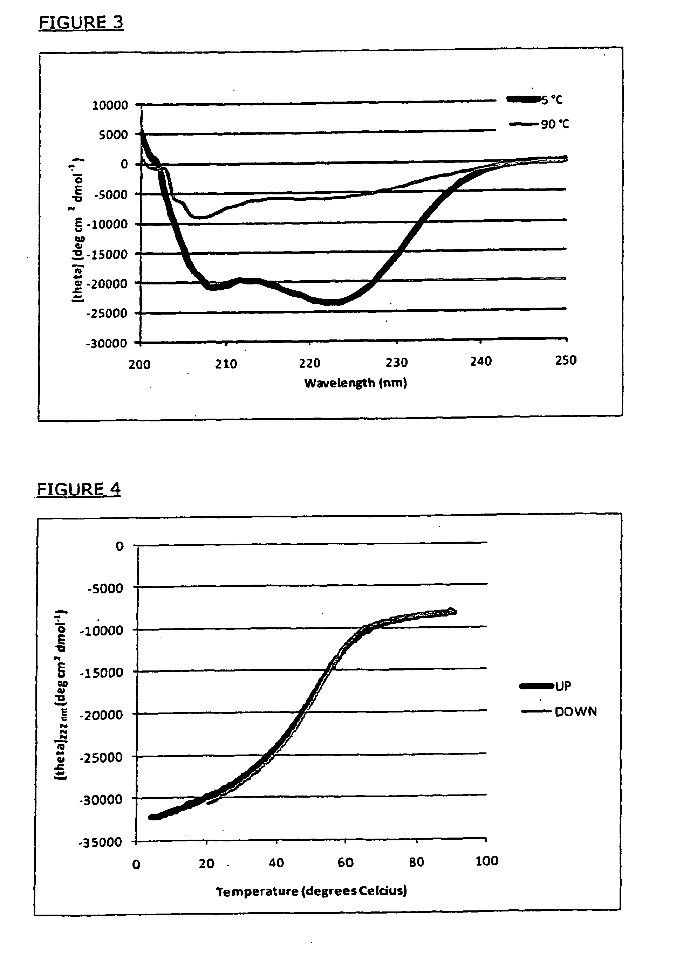

Figure 3 illustrates the thermal denaturation of a peptidic coiled coil, monitored by circular dichroism (CD). The CD spectrum of the peptide Ac-MSIEEIQKQQAAIQKQIAAIQKQIYRMTP-NH2 at 5 and 90 degrees Celcius is shown (black and gray curves, respectively). The peptide was dissolved at a concentration of 292 microM in 20 mM phosphate buffer (PBS), 150 mM NaCl, pH 7.2.

Figure 4 illustrates the reversible unfolding and folding of the peptide of Figure 3, as monitored by the CD signal at 222 nM as a function of temperature (UP and DOWN scans are shown).

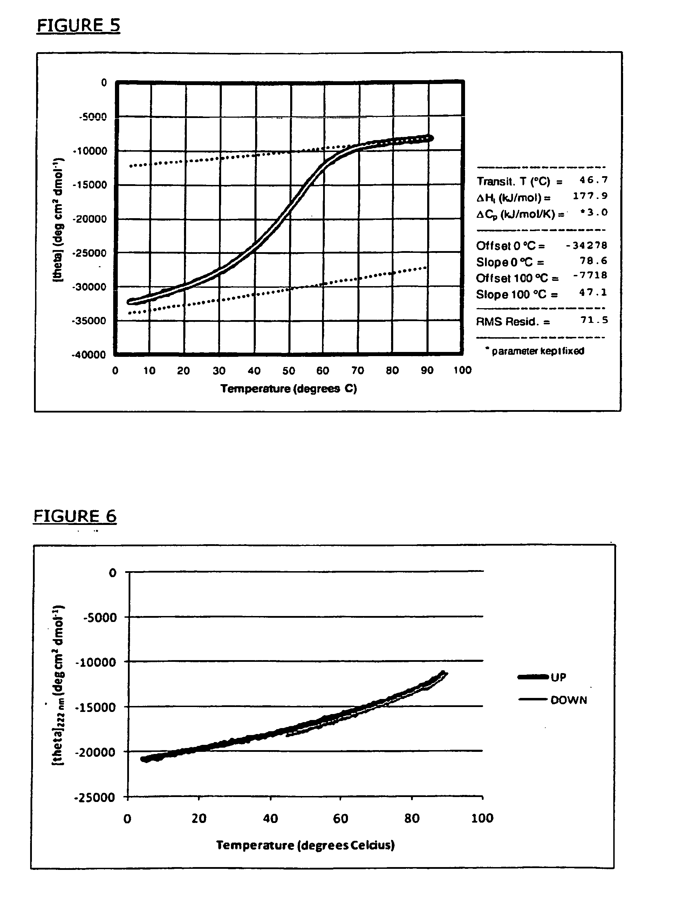

Figure 5 illustrates the further thermodynamic analysis of the thermal unfolding curve of Figure 4. The black curve represents experimental data taken from Figure 4, whereas the white curve represents the fitted curve. The theoretic (fitted) curve was obtained by the procedure explained in EXAMPLE 3. The fitted parameters (fitting results) are listed at the right in Figure 5. 'Transit. T' corresponds to $T_t$, but is expressed in degrees Celsius. The parameter 'delta $C_p$' was kept constant at 3.0 kJ mol$^{-1}$ K$^{-1}$. The parameters 'theta$_M$ ($T$)' and 'theta$_T$($T$)' were treated as linear functions of T, resulting in the dotted straight lines described by the respective offsets and slopes indicated at the right in the figure. 'RMS Resid.' refers to the root-mean-square of the differences between experimental and theoretic data points.

Figure 6 illustrates the CD thermal scan curve for a sample preparation of the Q2aI peptide under the same conditions as in Example 3. The Q2aI peptide has the amino acid sequence Ac-MSIEEIQKQIAAIQKQIAAIQKQIYRMTP-NH2. The results of an UP and DOWN scan are shown in black and gray, respectively.

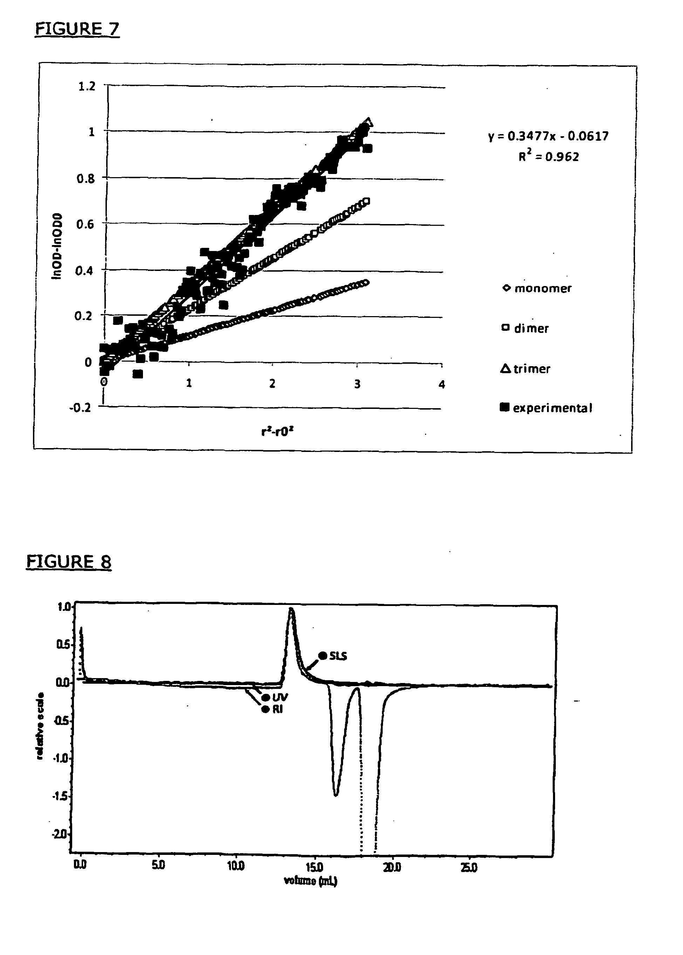

Figure 7 illustrates the analytical sedimentation equilibrium ultracentrifugation results for the Q2aI peptide of Figure 6. The sedimentation curve was obtained at 25000 rotations per minute (rpm). The figure shows the linearized optical density (OD) curve in comparison with the theoretical curves for monomeric, dimeric and trimeric complexes, as indicated by the labels.

Figure 8 illustrates the static light scattering results for the Q2aI peptide of Figure 6. 200 microliter peptide at 1 mg/ml in PBS was put on a Superdex 75 10/300 GL gel filtration column connected to ultra-violet (UV), refractive index (RI) and static light scattering (SLS) detectors. The signals (curves) from the three different detectors are labeled accordingly.

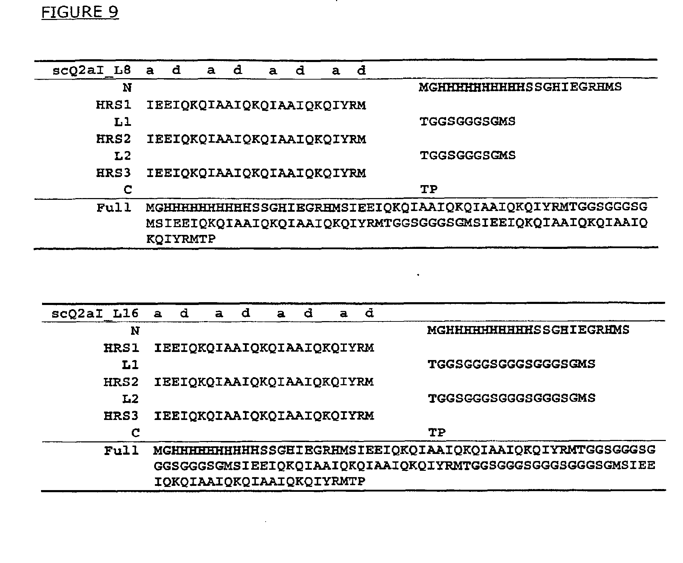

Figure 9 illustrates the amino acid sequences of two proteins forming specific embodiments of the present invention. These two proteins are referred to as 'scQ2aI_L8' and 'scQ2aI_L16', respectively. Their full amino acid sequences are listed at the bottom of each table panel, to the right of the label 'Full'. Specific segments within the same sequences are also shown on top, to facilitate identification of N- and C-terminal flanking segments (labeled 'N' and 'C', respectively), linker segments (labeled 'L1' and 'L2', respectively) and the actual heptad repeat sequences (labeled 'HRS1', 'HRS2' and 'HRS3'). Heptad a- and d-positions are provided at the top row to facilitate their identification within the heptad repeat sequences.

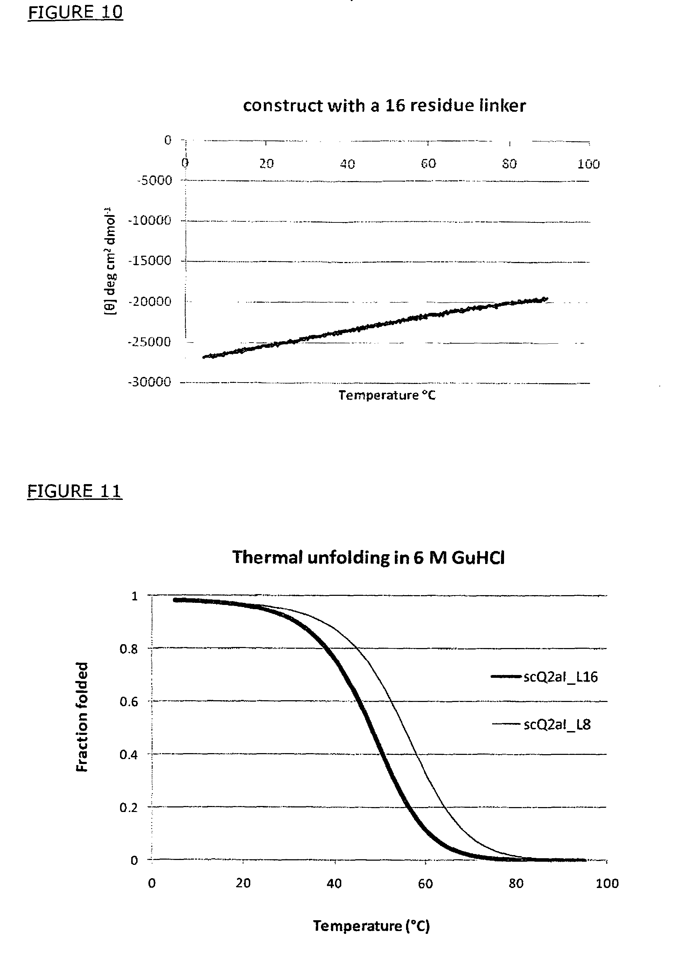

Figure 10 illustrates the CD thermoscan for the scQ2aI_L16 construct. The scan was recorded for this construct in 20 mM PBS, 150 mM NaCl, pH 7.2.

Figure 11 illustrates the thermal denaturation of scQ2aI_L16 and scQ2aI_L8 (labeled accordingly) in 6 M GuHCl recorded by CD at 222 nm in PBS buffer and at a protein concentration of about 30 μM. The thermoscans were fitted to a two-state transition model and converted to fraction folded protein.

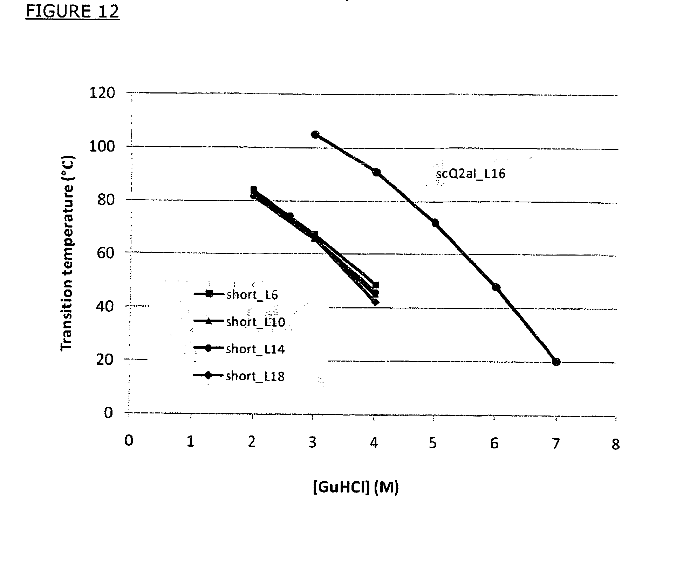

Figure 12 illustrates the transition temperatures of various constructs forming specific embodiments of the present invention, as a function of GuHCl (denaturant) concentration. Said constructs are referred to as 'scQ2aI_L16', 'short_L6', 'short_L10', 'short_L14' and 'short_L18', and the corresponding curves are labeled accordingly. The sequences of said constructs, a method for producing them, and experimental conditions are further detailed in EXAMPLE 5.

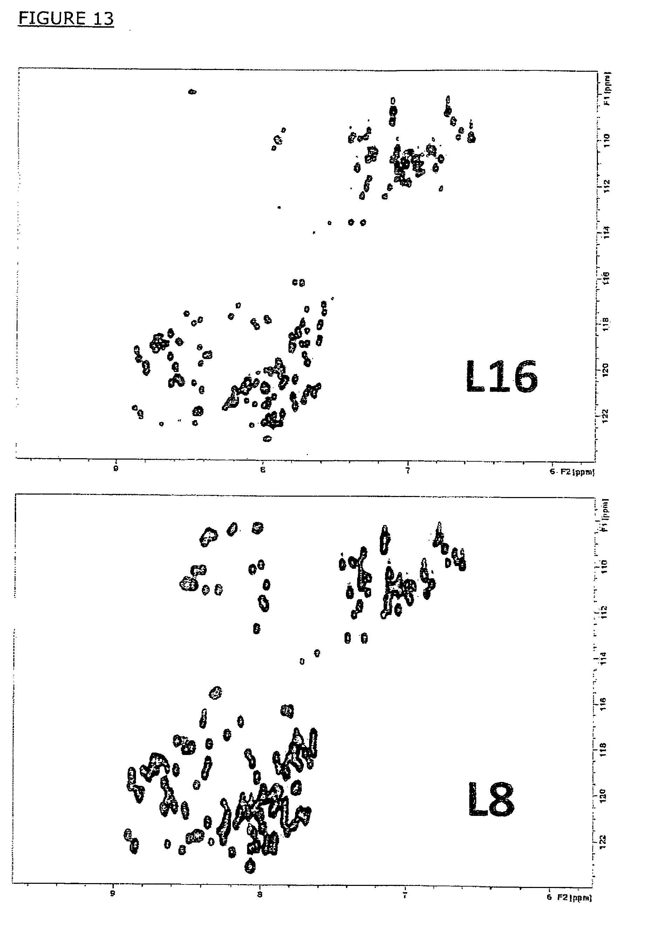

Figure 13 shows the $^{15}$N $^1$H HSQC NMR spectra for the constructs scQ2aI_L16 and scQ2aI_L8 (as labeled accordingly).

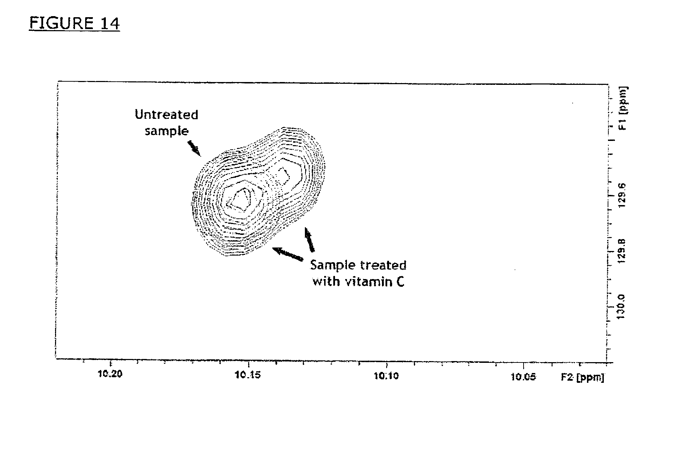

Figure 14 shows a zoom on the NMR spectrum of a spin-labeled tryptophan-cysteine double mutant of the scQ2aI_L16 construct, as explained in EXAMPLE 6. The spectrum was recorded on the untreated sample and on a vitamin C-treated sample (resonances labeled accordingly).

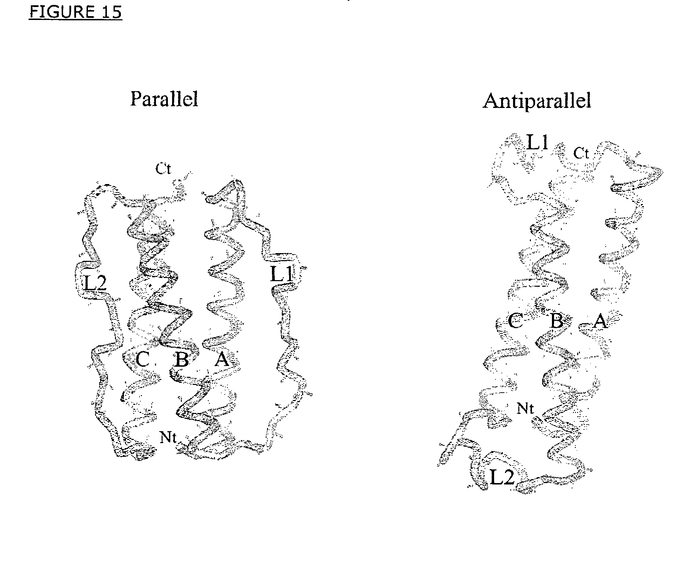

Figure 15 shows molecular models of parallel and antiparallel 3-stranded single-chain coiled coils (labeled accordingly). The models were prepared as explained in EXAMPLE 7. The three alpha-helices in each model are labeled 'A', 'B' and 'C' and represent heptad repeat sequences HRS1, HRS2 and HRS3 in said single-chain coiled coils, respectively. The labels 'L1' and 'L2' indicate the respective linker segments. 'Nt' and 'Ct' indicate the N- and C-termini of each construct, respectively.

DETAILED DESCRIPTION OF THE INVENTION

The term 'scaffold' is used within the context of the present invention to denote 'a specific, conformationally (structurally) and thermodynamically (thermally and chemically) stable proteinaceous (protein-like or protein) molecule with a specific, fixed (invariable, invariant) three-dimensional (3-D, tertiary) structure (spatial arrangement of constituting elements) consisting of one or more protein or proteinaceous polypeptide chains, the said structure being demonstrably tolerant to a variety of single and multiple amino acid substitutions at a variety of amino acid residue positions.

The notion 'tolerant to amino acid substitutions' is herein to be understood in the sense that the integrity (correctness) of the structure remains essentially unaltered upon performing said amino acid substitutions. It is evident that any amino acid substitution in a protein alters the 3-D structure to some extent, but such changes are in the public domain and herein considered non-essential if the protein backbone (main chain) of the mutated (substituted) 3-D structure remains structurally superimposable with the non-mutated (original, wild-type) structure; two structures are considered superimposable if at least 70% of the backbone atoms (excluding hydrogen atoms) of both structures can be superimposed with a root-mean-square (RMS) deviation of preferably less than 1 Ångström (1 Å), less preferably 2 Å or 3 Å. In cases wherein a structural superimposition is not feasible (e.g. if one of both 3-D structures is not available), then the notion 'tolerant to amino acid substitutions' is to be interpreted in the thermodynamic sense: a protein is considered tolerant to amino acid substitution(s) if the substitution(s) diminish the midpoint of thermal transition (transition temperature, Tt, melting temperature, Tm, unfolding temperature Tu) by preferably not more than 10 degrees Celsius (°C) compared to wild-type, less preferably by not more than 20 °C, or 30 °C, or 40 °C, or 50 °C, and in any case not to the extent that the substituted protein quantitatively unfolds at physiological temperature (37 °C). The property 'tolerant to a variety of substitutions at a variety of positions' is herein intended to mean tolerant to at least about 10 different amino acid residues at at least 5 different amino acid positions, more preferably at 10 positions, or 20 positions, most preferably at about 50% or more of all amino acid positions.

The essence of what is generally understood by a scaffold molecule is a molecule that acts as a carrier of chemical groups. Similarly, scaffold proteins (or, briefly, scaffolds) herein refer to protein or proteinaceous molecules that serve as carriers of amino acid side chains. They may also serve as carriers of other proteins, or fragments, domains or peptides that are attached to any of their termini (i.e., as part of a fusion construct), but this is not the intended meaning within the present context. Since amino acid side chains in a protein are attached to the main chain (backbone), the folded backbone formally constitutes the chemically purest form of a scaffold. However, pure protein backbones, with poly-glycine as the closest polypeptide analog, do not stably fold in solution, and therefore do not meet the requirements of a useful scaffold. Consequently, proteins that are partially or fully deprived of their side chains do not form the subject of the present invention. Instead, the present invention claims real-life proteins that adopt a given 3-D fold (in casu, a single-chain triple-stranded antiparallel alpha-helical coiled coil structure) and which do this in a thermodynamically stable manner, even after having undergone a substantial number of mutations. Thus, the term 'scaffold' refers to their structural and thermodynamical robustness, rather than to a carrier function.

The protein molecules of the present invention can be used as scaffolds, similarly to many other documented scaffolds (reviewed in Skerra [J Mol Recognit 2000, 13:167-187], Binz et al. [Nat Biotechnol 2005, 23:1257-1268], Hosse et al. [Protein Sci 2006, 15:14-27]). The notion 'used as a scaffold' essentially means that desired molecules (e.g., with a certain functionality) can be obtained (derived) from a preselected reference construct (reference scaffold). The derived molecules are typically amino acid-substituted or loop-substituted variants of the reference scaffold.

Non-immunoglobulin protein-based (proteinaceous) scaffold molecules are considered in the field as a 'next-generation' class of compounds for molecular recognition. They are mostly derived from natural protein molecules which have been selected on basis of preferred physico-chemical properties and available experimental data. Examples of this class of compounds are listed by Hosse et al. [Protein Sci 2006, 15:14-27] and by Binz et al. [Nat Biotechnol 2005, 23:1257-1268].

The present invention discloses a particular type of non-immunoglobulin protein molecules that have excellent properties for use as protein scaffolds. Because of their high stability and structural robustness, large libraries (scaffold-based libraries, scaffold libraries) of molecules with essentially the same tertiary structures and slightly different sequences can be constructed. Alternatively, surface residues can be varied by making use of standard protein engineering methods. Making use of the skilled person's knowledge, appropriate selection methods can be applied for the purpose of identifying variants (scaffold derivatives, specific molecular compounds) with highly desired binding properties (e.g., affinities and specificities) similar to immunoglobulins.

Protein-based scaffold molecules have been ascribed numerous advantages over immunoglobulins including, for example, their relatively small size, high structural stability and absence of post-translational modifications. These features considerably facilitate their synthesis, purification and storage. Moreover, high-affinity compounds can be generated without the need to proceed via an immunization step. The protein scaffolds of the present invention embody all of aforementioned features, thereby rendering them particularly well-suited for scaffold-based applications.

The present invention relates to a particular type of protein-based scaffold that is largely insensitive to substitution of surface residues and standard protein engineering actions. All embodiments of the present invention relate to a specific type of protein structure (3-D structure, tertiary structure, fold) that has so far not been exploited as a highly mutatable protein scaffold, in casu, a single-chain triple-stranded antiparallel alpha-helical coiled coil structure.

The proteins of the present invention have a broad spectrum of possible applications, largely comparable to those of immunoglobulins. More concretely, specific scaffold-derived mutants may be usable as therapeutic compounds (e.g., inhibitors), detection probes (e.g., detection of a recombinant protein) and purification probes (e.g., in affinity chromatography), as detailed hereinafter.

The protein molecules of the present invention may be suitable as therapeutic compounds. More specifically, they may interfere with (influence, modify) biological processes through impeding (blocking, inhibiting) natural chemical reactions or natural molecular recognition events, or through creation of non-natural molecular recognition events. Instances of biological interference include, without limitation, blocking of human receptors, binding to pathogenic species, and binding to disease- or disorder-related proteins. Such type of biological interference is typically intended to curate severe diseases or disorders. These applications belong to the field of therapeutic research and development. Current therapeutic treatments are generally based on pharmacological or biotechnological compounds, the latter including either immunoglobulin(-derived) or non-immunoglobulin compounds. The production, purification, testing and optimization of both types of biotechnological compounds is generally labor-intensive, riskful and expensive. Accordingly, there is a need for new biotechnological compounds with specific biological activity, as well as improved methods for the production, purification, testing and optimization of such compounds.

The protein molecules of the present invention may be suitable as detection probes. Instances wherein specific probe molecules (probes) are applied to detect the presence of an analyte of interest (target analyte) in a given sample of interest (study sample), include, without limitation, experimental analyses of samples of human, animal, plant, bacterial, viral, biotechnological or synthetic origin. Such samples typically contain biomolecules (e.g., polypeptides, polynucleotides, polysaccharides, hormones, vitamins or lipids, or derivatives thereof) that can interact specifically with a selected probe molecule. The latter interaction typically gives rise to a characteristic (e.g., spectroscopic or radioactive) signal, indicative of the presence of said target analyte in said study sample. These applications belong to the field of analytical research and development. The number of combinations of different types of probes and targets that are effectively used in medical and biotechnological applications is virtually unlimited. In view of the continuous evolution in these areas, there is an ongoing need for new analytical tools (e.g., probes) with desired physico-chemical properties (e.g., specificity, affinity, stability, solubility), as well as improved methods for the production, purification, testing and optimization of such compounds.

The protein molecules of the present invention may be suitable for purification applications. Instances wherein specific ligand molecules (ligands) are applied to retain (extract, isolate, purify, filter) other molecules of interest (targets, target analytes) in a given sample of interest (crude sample) include, without limitation, samples of human, animal, plant, bacterial, viral, biotechnological or synthetic origin containing biomolecules (e.g., polypeptides, polynucleotides, polysaccharides, hormones, vitamins or lipids, or derivatives thereof) that can interact (associate) with high specificity with selected ligand molecules, where the latter are separated, or can be separated, from the crude sample (e.g., by attachment onto a solid support or by precipitation), for the purpose of co-separating the target molecules from the crude sample. These applications belong to the field of purification technology. More specific examples of purification methods include affinity chromatography and immunoprecipitation. In view of the continuous evolution in these areas, there is an ongoing need for new ligands for purification with desired physico-chemical properties (e.g., specificity, affinity, stability, solubility), as well as improved methods for the production, purification, testing and optimization of such compounds.

The protein scaffold molecules of the present invention fold into an alpha-helical coiled coil structure. The alpha-helical coiled coil forms a special type of 3-D structural framework (structural motif, fold). The coiled coil fold occurs in a wide variety of proteins including motor proteins, DNA-binding proteins, extracellular proteins and viral fusion proteins (e.g., Burkhard et al. [Trends Cell Biol 2001, 11:82-88]). It has been estimated that 3 to 5 %, or more, of all amino acids in natural proteins are part of a coiled coil structure [Wolf et al., Protein Sci 1997, 6:1179-1189].

Coiled coils have been functionally characterized as folding (assembly, oligomerization) motifs, i.e., formation of a coiled coil structure drives in many instances the non-covalent association of different protein chains. Coiled coils have been structurally characterized as 2-, 3-, 4- or 5-stranded assemblies of alpha-helices arranged in parallel, antiparallel or mixed topologies (e.g., Lupas [Trends Biochem Sci 1996, 21:375-382]. The helices are slightly wrapped (coiled, wound) around each other in a left- or right-handed manner, termed supercoiling. All embodiments of the present invention exclusively relate to triple-stranded (3-stranded, trimeric) coiled coil structures.

Alpha-helical coiled coils have been further characterized at the level of their amino acid sequences, in that, each helix is constituted of a series of heptad repeats. A heptad repeat (heptad unit, heptad) is a 7-residue sequence motif which can be encoded as HppHppp, and wherein each 'H' represents a (potentially different) hydrophobic residue and each 'p' is a (potentially different) polar residue. Occasionally (infrequently), p-residues are observed at H-positions, and vice versa. A heptad repeat of the invention is encoded by the patterns a-b-c-d-e-f-g (abcdefg) or d-e-f-g-a-b-c (defgabc), in which case the indices 'a' to 'g' refer to the conventional heptad positions at which typical amino acid types are observed. By convention, indices 'a' and 'd' denote the positions of the core residues (central, buried residues) in a coiled coil. The typical amino acid types that are observed at core a- and d-positions are hydrophobic amino acid residue types; at all other positions (non-core positions), predominantly polar (hydrophilic) residue types are observed. Thus, conventional heptad patterns 'HppHppp' match with the pattern notation 'abcdefg' ('HpppHpp' patterns match with the pattern notation 'defgabc', this notation being used for coiled coils starting with a hydrophobic residue at a d-position). All embodiments of the present invention include at least 2, preferably 3 or more consecutive (uninterrupted) heptad repeats in each alpha-helix of the coiled coil structure. Each series of consecutive heptad repeats in a helix is denoted a 'heptad repeat sequence' (HRS). The start and end of a heptad repeat sequence is preferably determined on the basis of the experimentally determined 3-dimensional (3-D) structure, if available. If a 3-D structure is not available, the start and end of a heptad repeat sequence is preferably determined on the basis of an optimal overlay of a $(HppHppp)_n$ or $(HpppHpp)_n$ pattern with the actual amino acid sequence, where 'H' and 'p' denote hydrophobic and polar residues, respectively, and where 'n' is a number equal to or greater than 2. Then the start and end of each heptad repeat sequence is taken to be the first and last hydrophobic residue at an a- or d-position, respectively. Conventional H-residues are preferably selected from the group consisting of valine, isoleucine, leucine, methionine, phenylalanine, tyrosine, tryptophan, histidine, glutamine, threonine, serine and alanine, more preferably from the group consisting of valine, isoleucine, leucine and methionine, and most preferably isoleucine. Conventional p-residues are preferably selected from the group consisting of glycine, alanine, cysteine, serine, threonine, histidine, asparagine, aspartic acid, glutamine, glutamic acid, lysine and arginine. In case this simple method does not permit unambiguous assignment of amino acid residues to a heptad repeat sequence, a more specialized analysis method can be applied, such as the COILS method of Lupas et al. [Science 1991, 252:1162-1164; http://www.russell.embl-heidelberg.de/cgi-bin/coils-svr.pl].

Coiled coils have been thermodynamically characterized as follows. When the sequence folds into an alpha-helix, the hydrophobic residues (H) form a hydrophobic seam, whereas the polar residues (p) form a polar face. The hydrophobic seams of different alpha-helices, when associated into a coiled coil, form a central hydrophobic core (center, interior, inner part). Formation of this core, in combination with orientation of the polar faces toward solvent, is assumed to provide the main thermodynamic driving force required for stable association, although certain non-core residues may enhance stability as well. All embodiments of the present invention relate to triple-stranded coiled coil structures consisting of at least two heptad repeats per alpha-helix and wherein the H-residues of the heptad repeats form the hydrophobic core and, as such, provide the main thermodynamic driving force for folding of the structure.

Peptidic (non-single-chain) 3-stranded coiled coils can exhibit a high thermal stability in spite of their dependence on oligomerization and, hence, high concentration dependence. For example, the Ile-zipper of Suzuki et al. [Protein Eng 1998, 11:1051-1055] was shown to have a melting (unfolding, transition) temperature exceeding 80°C. Similarly, Harbury et al. [Science 1993, 262:1401-1407; Nature 1994, 371:80-83] designed a GCN4-derived triple-stranded coiled coil, named GCN4-pII, which was found stable in the crystal and in solution. Further, heterotrimeric parallel coiled coils were also designed with success [Nautiyal and Alber, Protein Sci 1999, 8:84-90]. The main rules for peptides to assemble into trimeric parallel configurations are also grossly known [Yu, Adv Drug Deliv Rev 2002, 54:1113-1129]. Further, international application PCT/EP2008/061886 has claimed peptidic 3-stranded coiled coils under the form of a non-natural, thermodynamically stable, proteinaceous scaffold. The molecules of the present invention also comprise a 3-stranded coiled coil structure, but they fundamentally differ from peptidic coiled coils (which form trimeric complexes) in that, they are made of a single amino acid chain that folds as a monomeric protein.

While the previous may suggest that the design of 3-stranded parallel coiled coils is relatively straightforward, many studies have reported serious difficulties. For example, a coiled coil that was designed as a parallel dimer was observed in the crystal structure as an antiparallel trimer [Lovejoy et al., Science 1993, 259:1288-1293]. Further, the requirement of a trigger sequence for enhancing the folding kinetics has been a matter of debate [Yu, ibid]. In addition, the thermal unfolding process does not always follow a simple two-state mechanism [Dragan and Privalov, J mol Biol 2002, 321:891-908] and the assembly (folding) process is occasionally very slow [Dragan et al., Biochemistry 2004, 43:14891-14900]. Accordingly, in view of the many unexpected results obtained by skilled researchers despite abundance of experimental data on parallel coiled coils, it can be concluded that the design and application of even parallel alpha-helical coiled coil molecules is absolutely not obvious. Consequently, the development of antiparallel coiled coils can be envisaged as being still more complicated.

The inventors initially contemplated the use of peptidic triple-stranded coiled coil scaffolds, while at the same time attempting to find a practical solution to the inherent disadvantages of such complexes, which have to trimerize first in solution before adopting the proper (i.e. intended, functional) fold. Such solution was eventually found under the form of a single-chain version of a trimer, wherein the C-terminal end (C-terminus) of a first constituting alpha-helix is connected (joined, linked) to the N-terminal end (N-terminus) of a second alpha-helix, and the C-terminal end of the latter to the N-terminal end of a third alpha-helix. According to the terminology of Harris et al. [J Mol Biol 1994, 236:1356-1368], connections between parallel helices are called 'overhand' (or 'long') connections, and between antiparallel helices they are called 'underhand' (or 'short') connections. In the embodiments of the present invention, connections between consecutive alpha-helices are realized through the usage of structurally flexible linker fragments, giving rise to constructs wherein three alpha-helices are linked together by two flexible linkers. All embodiments of the present invention belong to this type of arrangement. The molecules of the present invention can therefore be formally written as a sequence of the formula HRS1-L1-HRS2-L2-HRS3, wherein HRS1, L1, HRS2, L2 and HRS3 represent amino acid sequence fragments that are covalently and consecutively interconnected in the order as indicated in the said formula, and wherein fragments HRS1, HRS2 and HRS3 are heptad repeat sequences as described supra, and wherein fragments L1 and L2 are structurally flexible linker sequences.

Flexible linker fragments are frequently used in the field of protein engineering to interconnect different functional units, e.g. in the creation of single-chain variable fragment (scFv) constructs derived from antibody variable light (VL) and variable heavy (VH) chains. At present, the application of flexible linker fragments in combination with trimeric coiled coil structures, for the purpose of creating a single-chain yet triple-stranded coiled coil scaffold structure has not been disclosed nor anticipated in the public domain. It is also remarked that there is no contradiction in the formulation 'single-chain yet triple-stranded' because 'single-chain' refers to the full amino acid sequence, whereas 'triple-stranded' is the common term to denote that the coiled coil structure consists of three individual alpha-helical strands (chain fragments). All embodiments of the present invention comprise exactly two flexible linker segments (fragments) within the context of a 3-stranded coiled coil structure. The linker segments are not necessarily identical in length or amino acid sequence. Yet, to enhance the probability that they are conformationally flexible in solution, they are preferably and predominantly composed of polar amino acid residue types. Typical (frequently used) amino acids in flexible linkers are serine and glycine. Less preferably, flexible linkers may also include alanine, threonine and proline. Still less preferred (because of the increasing risk of undesired interactions) is the incorporation of cysteine, histidine, asparagine, aspartic acid, glutamine, glutamic acid, lysine and arginine, or non-natural derivatives thereof, in combination with the said more preferred amino acids.

A preferred and simple method to distinguish the linker fragments from the heptad repeat sequences is to first determine the latter by any of the methods described supra, and then to include the remaining amino acid fragments in the linkers. This method applies both to the case wherein there exists no experimentally determined 3-D structure of the protein molecule and to the case wherein there does exist one or more such structures. If such experimentally determined structure(s) would give rise to uncertainty or ambiguity concerning the structurally flexible state of any of the linkers, than the notion 'flexible linker' is to be interpreted merely as a fragment that is able to connect (link, bridge) between two heptad repeat sequences, and not as a structurally dynamic or mobile fragment.

The use of flexible linkers in the present invention is primarily intended to interconnect the alpha-helical fragments for the purpose of creating a linear amino acid sequence (single-chain construct). While this is technically straightforward, an important aspect that has to be considered is the length (number of amino acid residues) of each linker. For parallel coiled coils wherein the helices comprise the same number of residues, the distance in 3-D space from the end (C-terminus) of a given alpha-helix to the beginning (N-terminus) of an adjacent alpha-helix (overhand connection) can be roughly calculated by the formula 'number of residues per alpha-helix, multiplied by 1.5 Ångström'. The distance that can be bridged by a linker in extended conformation can be roughly calculated by the formula 'number of residues in the linker fragment, multiplied by 3.0 Angstrom'. Hence, as a rule, a linker must have at least half of the number of residues per alpha-helix to enable overhand connection in a relaxed manner. (Exceptions to this rule apply when the helices are of different length or when the helix-to-linker turns are not easily made: in such cases, a small number of additional linker residues is preferably added.)

Importantly, said rule provides a practical way to calculate the minimum linker length needed for an overhand connection between alpha-helical elements in parallel configuration, and not a method to impose parallel orientation. The conformation of a flexible linker in solution will, or is at least intended to be essentially random in structure and dynamic in behavior (i.e., structurally variable in time). Hence, a linker 'of sufficient length' will permit, but not impose, parallel folding. Reversely, a linker 'of insufficient length' ('too short linker') will not permit parallel folding and therefore induce either unfolding or formation of an alternative fold (provided the latter is stable itself). One such possibility of an alternative fold is an antiparallel coiled coil structure: the requirements for linkage between antiparallel helices (underhand connection) are topologically very complex, but are generally less restrictive. In other words, a linker that is significantly too short to bridge the distance between alpha-helices in parallel orientation may very well permit antiparallel folding. Importantly, the latter does not imply that such short linker is required for, or will necessarily induce, antiparallel folding - the latter essentially depends on the possibility of the formation of a physically and thermodynamically stable core in antiparallel mode, possibly further enhanced by additional favorable interactions between non-core residues.

Since it has been observed that trimeric coiled coil structures fold, with rare exceptions, in parallel orientation, it is unlikely that the same sequences can also adopt a stable antiparallel fold. The latter is of specific relevance for the present invention, because the inventors have generated and characterized single-chain triple-stranded coiled coil structures that were provided with linkers that are significantly too short for parallel folding, while yet the molecules folded with full preservation of alpha-helical content and with negligible effects on the transition temperature in thermal unfolding experiments (see EXAMPLE 5). Based on these experiments, it was concluded that these constructs (and possibly also those with long linkers) presumably adopt an antiparallel fold.

To test whether antiparallel folding is structurally feasible, the inventors have attempted to generate 3-D models of a single-chain trimeric coiled coil wherein the second alpha-helix ('B') is antiparallel to the first ('A') and third ('C'). Unexpectedly, credible models with regular 'knobs-into-holes' packing could be generated by standard protein modeling operations (see EXAMPLE 7). All core-forming side chains could be placed in their most relaxed rotameric conformation. Interestingly, conventional heptad a-positions of the antiparallel B-helix pack onto d-residues of the A- and C-helices (d-layers). In this way, the B-helix interacts with A and C over its entire length, suggesting that all heptad core positions contribute to the stability of the fold. While this does not prove that antiparallel folding is the case, the modeling results suggest that it is at least structurally feasible, in contrast to the original assumptions.

A similar unexpected observation was made by Lovejoy et al. [Science 1993, 259:1288-1293] for 'Coil-Ser', a peptide that was designed to form a double-stranded parallel coiled coil, but actually assembled into a triple-stranded coiled coil. This structure was stabilized by a distinctive, unintended hydrophobic interface consisting of eight layers (each a-layer within the parallel helices was found to be associated with a d-residue from the antiparallel helix, and each d-layer was associated with an a-residue; the layers were termed 'a-a-d' and 'd-d-a', respectively). In another study by Holton and Alber [Proc Natl Acad Sci USA 2004, 101:1537-1542], a GCN4 leucine zipper Ala-mutant also switched from the default parallel dimer configuration into an antiparallel trimer configuration. This structural switch was found due to the avoidance of creating cavities in the core. The same arrangement into alternating a-a-d and d-d-a layers was found as in the Holton and Alber study. Importantly, both of these studies related to coiled coils having a core formed by leucine residues ('Leu-zippers'), whereas the present inventors observed an antiparallel orientation for coiled coils having a core formed by isoleucine residues ('Ile-zippers'); never before have Ile-zippers been found to form antiparallel 3-stranded coiled coils. Second, both of the said studies related to peptidic coiled coils, whereas the molecules of the present invention exclusively relate to single-chain coiled coils; never before have antiparallel 3-stranded coiled coils been made in the form of single-chain molecules (single-chain format). It is not known whether the antiparallel orientation of these protein molecules is due to the presence of the linker fragments, or to their specific amino acid sequences, or to any other reason, or to a combination of reasons. In any case, the 3-D models of the molecules of the present invention, as well as the crystallographic structures described in the cited studies by Lovejoy et al. [*ibid*] and Holton and Alber [*ibid*], are all true coiled coil structures with regularly packed core residues in regularly spaced layers. This distinguishes them from ordinary three-helix bundles.

Triple-stranded antiparallel coiled coil structures are not to be confused with ordinary three-helix bundles: there are plentiful examples of associations (bundles) of three alpha-helices that are not regular coiled coils. Bundles of alpha-helices can be observed in a large number of mainly alpha-helical proteins, and bundles of three mutually interacting helices can often be discerned within such proteins. Triple-stranded coiled coils are evidently also bundles of three alpha-helices, but in order for a 3-helix bundle to be a coiled coil, a number of additional conditions need to be fulfilled. First, it is required that all three helices mutually interact with each other, which excludes topologies wherein only two of the three possible pairs of helices are in contact with each other (non-cohesive topologies). Second, there must be an appropriate degree of supercoiling (i.e., wrapping of the helices around each other). The primary determinant of supercoiling is the angle between each pair of helices (interhelical angle, helix-helix interaction angle, crossing angle). For parallel alpha-helices, this angle can vary from small negative values for 'right-handed' supercoiling, typically in the range of about -10 degrees to 0 degrees, to positive values for 'left-handed' supercoiling, typically in the range of about 20 degrees to 0 degrees. For antiparallel alpha-helices, 180 degrees is to be subtracted from the said values. Topologies with a too high angle, the latter set at 40 degrees in absolute value, are not considered as coiled coils. Third, there must be discernible heptad repeats within each of the interacting alpha-helices (as defined supra). True coiled coils comprise at least 2, preferably at least 3 heptad repeats in each alpha-helix. Fourth, the alpha-helices must be tightly packed against each other by way of their side chains interacting in a knobs-into-holes fashion, as illustrated for parallel dimeric, trimeric and tetrameric coiled coils in Harbury et al. [Nature 1994, 371:80-83] and for antiparallel trimeric coiled coils in Lovejoy et al. [Science 1993, 259:1288-1293]. Walshaw et al. [J Struct Biol 2003, 144:349-361] describe more sophisticated rules and a method to distinguish true coiled coils from multi-helix assemblies.

In addition to the foregoing, the protein molecules of the present invention exist as isolated proteins and do not require additional associated alpha-helices (or other protein fragments) for their stable folding in solution, as is the case for certain classes of complex coiled coil assemblies listed in the 'CC+ database of coiled coils' [http://coiledcoils.chm.bris.ac.uk/ccplus/search/periodic table].

Further, the coiled coil structures of the present invention contain no irregularities in their heptad repeat sequences (i.e., stammers or stutters), meaning that they have the standard 3-4 spacing between consecutive core residues (at conventional heptad 'a' and 'd' positions) along the sequence.

As far as it is possible to measure (i.e., if a 3-D structure can be obtained), the molecules of the present invention also have a high degree of structural symmetry, in that, they have repeated, regularly spaced layers of core 'a' residues (a-layers) and core 'd' residues (d-layers) within the two parallel alpha-helices that exist within the antiparallel coiled coil fold. Since the core residues form the primary determinants of the type of folding, structural symmetry can also be discerned, and even imposed, on basis of the amino acid sequence, i.e., by appropriate selection of core amino acid residues. Such structural symmetry is important for developing non-natural (designed) coiled coil molecules, because it renders the design task manageable (irregular structures cannot be designed de novo). Moreover, the creation of structural symmetry by way of introducing symmetry at the level of the core residues considerably enhances the likelihood of folding into highly stable, regular coiled coils.

One possibility to ensure formation of regular a-and d-layers is by avoiding selection of bulky aromatic residues (tryptophan, tyrosine, phenylalanine) and tiny residues (glycine, alanine) at core positions. Another way to promote regular a- and d-layers is by selecting hydrophobic core residues of moderate size, such as isoleucine, leucine, methionine and valine. Yet another way to obtain regular a- and d-layers is by selecting the same amino acid residues in consecutive layers of the core (e.g., isoleucine at all a-layer positions). Yet another way to obtain regular core layers is by selecting the same amino acid residues at equivalent core positions in adjacent alpha-helices (e.g., isoleucine at the first heptad a-position in both the first and the third alpha-helix, these helices forming the parallel helices of the coiled coil structure). In general, the higher the amino acid sequence symmetry at the core positions, the higher will be the chance that designed molecules will fold as desired. Hence, the molecules of the present invention include at least some, preferably a fair, most preferably a high degree of sequence symmetry and, thereby, structural symmetry.

The existence or lack of symmetry forms an adequate discriminator between the molecules of the present invention and known 3-helix bundles which do not form embodiments of the invention. In nature, highly symmetric coiled coils are only observed as oligomers and never as single-chain molecules. (The underlying reasons for this observation are complex and intriguing, but are of little importance here.) Reversely, natural single-chain 3-helix bundles are not only very rare (they usually appear as small antiparallel domains in larger proteins or complexes), they are also markedly devoid of internal symmetry.

One of the closest examples of prior art on antiparallel 3-helix bundles is found in the PDB structure of the human GGA1 GAT domain [Zhu et al., EMBO J 2004, 23:3909-3917; PDB code: 1X79]. Residues 210-302 of GGA1 GAT domain form an antiparallel three-helix bundle motif which might perhaps be confused with the antiparallel coiled coil structures of the present invention. The first alpha-helix in this bundle runs largely parallel with the third helix, while the second helix is oriented antiparallel to these. Packing is relatively tight and occurs in a knobs-into-holes fashion. However, the two parallel helices are devoid of packing symmetry, as can be observed from the absence of a- and d-layers in the structure, and the absence of structurally similar heptad core residues in the amino acid sequences of helices 1 and 3: large (1 arginine, 1 tyrosine) and small (2 alanines) interdigitate with a mixture of aliphatic core residues (valine, isoleucine, leucine). Moreover, the crystallographers do not denote the GAT domain a coiled coil (but a 3-helix bundle), while they do classify the bound rabaptin5 ligand as a (dimeric) coiled coil. Other examples of non-coiled coil 3-helix bundles include the B, E and Z domains in Staphylococcal protein A and the tertiary structure of villin headpiece.

The specific type and format of the coiled coil-forming molecules of the present invention are not observed in nature, which is one of the reasons why they are preferably referred to as 'non-natural'.

The present invention primarily relates to, and a preferred embodiment of the present invention includes, an isolated single-chain protein being represented by the formula HRS1-L1-HRS2-L2-HRS3, wherein HRS1, L1, HRS2, L2 and HRS3 represent amino acid sequence fragments that are covalently interconnected <page 32a> said protein spontaneously folding in aqueous solution by way of the HRS1, HRS2 and HRS3 fragments forming a triple-stranded, antiparallel, alpha-helical coiled coil structure, and wherein

each of HRS1, HRS2 and HRS3 is independently a heptad repeat sequence that is characterized by a n-times repeated 7-residue pattern of amino acid types, represented as (a-b-c-d-e-f-g-)n or (d-e-f-g-a-b-c-)n and a C-terminal partial heptad repeat ending with a heptad core residue located at an a- or d-position, wherein the pattern elements 'a' to 'g' denote conventional heptad positions at which said amino acid types are located and n is a number equal to or greater than 2, and at least 50%, 70%, 90%, or 100% of the conventional heptad positions 'a' and 'd' are occupied by amino acids selected from the group consisting of valine, isoleucine, leucine, methionine, phenylalanine, tyrosine, tryptophan, histidine, glutamine, threonine, serine, alanine derivatives thereof, wherein at least 50%, 70%, 90%, or wherein 100% of the conventional heptad positions 'b', 'c', 'e', 'f' and 'g' are occupied by amino acids selected from the group consisting of glycine, alanine, cysteine, serine, threonine, histidine, asparagine, aspartic acid, glutamine, glutamic acid, lysine, arginine or derivatives thereof, the resulting distribution of amino acid types enabling the identification of said heptad repeat sequences, and

each of L1 and L2 is independently a linker consisting of 1 to 30 amino acid residues, this linker including any amino acid residue that cannot be unambiguously assigned to a heptad repeat sequence, and

L1 and L2 have an amino acid composition comprising at least 50% amino acids selected from the group consisting of glycine, alanine, serine, threonine, proline or derivatives thereof.

The aforementioned property 'isolated' essentially relates to the requirement that the proteins of the present invention form stable structures without the need to be further associated with ligands (e.g., other proteins, peptides, nucleic acids, carbohydrates ions, etc), or be embedded within a larger protein context (i.e., within a fusion construct or as a domain), as also explained supra.

The aforementioned property 'non-natural' essentially relates to the requirement that the proteins of the present invention are not observed in nature, as natural proteins, or as naturally occurring protein domains. To distinguish them from natural proteins or domains, the percentage amino acid sequence identity amounts to preferably less than 90%, more preferably less than 80%, most preferably less than 70%. The term 'non-natural' also refers to the fact that the proteins are designed, or conceived, preferably on a rational basis by humans.

The aforementioned property 'single-chain' essentially relates to the fact that the proteins of the present invention are made of a single amino acid chain (polypeptide chain), and not of oligomeric (dimeric, trimeric, etc) assemblies. This implies that they can be isolated as monomers in solution. The latter, however, does not exclude the possibility that they can interact with (associate with, form complexes with, bind to) other molecules, biological entities, or non-biological materials in vitro or in vivo.

The aforementioned property 'protein' essentially means a polypeptide composed of amino acids (amino acid residues, optionally non-natural or derivatized amino acids) arranged in a linear chain and folded in solution (aqueous solution, water-rich medium) into a globular form.

The aforementioned term 'spontaneously' essentially means in a reasonable (non-extreme) time, under reasonable conditions, by itself.

The aforementioned term 'folding' essentially means the formation of a globular (compact, 'globe-like') fold, this formation being characterized and driven by intra-chain, interatomic interactions.

The aforementioned terms 'triple-stranded', 'antiparallel', 'coiled coil structure', 'heptad repeat sequence', 'pattern', 'conventional heptad positions', 'predominantly occupied by', 'hydrophobic amino acid types', 'hydrophilic amino acid types', 'linker' and 'unambiguously assigned to a heptad repeat sequence' have the meaning as explained elsewhere in this document. They are chosen so as to maximally conform to common terminology in the field.

Related to this invention is also a method for the production of said protein. Such a method entails for example the expression of said protein in a bacterial host, as described in EXAMPLE 5. Alternatively, expression of said protein can be carried out in eukaryotic systems such as yeast or insect cells. Alternatively, the small size of said protein allows its production via chemical synthesis, using process steps well known in the art.

The present invention relates to a single-chain antiparallel coiled coil protein wherein at least 50%, preferably at least 70%, at least 90%, or wherein 100% (all) of the conventional heptad positions 'a' and 'd' are occupied by amino acids selected from the group consisting of valine, isoleucine, leucine, methionine, phenylalanine, tyrosine, tryptophan, histidine, glutamine, threonine, serine, alanine or derivatives thereof. The preferred percentage of said amino acids at said conventional heptad positions depends on the level of risk one is prepared to take in the design of said protein. A percentage below 50% is considered to form a too high risk for the correctness of the fold.

Another preferred embodiment of the present invention relates to a single-chain antiparallel coiled coil protein wherein at least 50%, 70%, 90%, or wherein 100% of the conventional heptad positions 'a' and 'd' are occupied by amino acids selected from the group consisting of valine, isoleucine, leucine, methionine or derivatives thereof. Since the latter amino acids correspond to more standard (more frequently observed) coiled coil core residues, this embodiment is preferred over the previous.

Another preferred embodiment of the present invention relates to a single-chain antiparallel coiled coil protein wherein at least 50%, 70%, 90%, or wherein 100% of the conventional heptad positions 'a' and 'd' are occupied by isoleucines. Since the initial discovery of said single-chain antiparallel coiled coil protein was made with constructs having isoleucine residues at conventional heptad positions 'a' and 'd', this embodiment is preferred over the previous.

The present invention relates to a single-chain antiparallel coiled coil protein wherein at least 50%, 70%, 90%, or wherein 100% of the conventional heptad positions 'b', 'c', 'e', 'f' and 'g' are occupied by amino acids selected from the group consisting of glycine, alanine, cysteine, serine, threonine, histidine, asparagine, aspartic acid, glutamine, glutamic acid, lysine, arginine or derivatives thereof. The preferred percentage of said amino acids at said conventional heptad positions depends on the level of risk one is prepared to take in the design of said protein. A percentage below 50% is considered to form a too high risk for the correctness of the fold and for the solubility of the protein.

The present invention relates to a single-chain antiparallel coiled coil protein wherein L1 and L2 have an amino acid composition comprising at least 50%, 70%, 90%, or comprising 100% amino acids selected from the group consisting of glycine, alanine, cysteine, proline, serine, threonine, histidine, asparagine, aspartic acid, glutamine, glutamic acid, lysine, arginine or derivatives thereof. The preferred percentage of said amino acids within the linkers depends on the level of risk one is prepared to take in the design of said protein. A percentage below 50% is considered to form a too high risk for the correctness of the fold, for the solubility of the protein, and for its possible function (e.g., specific binding to a given target).

Another preferred embodiment of the present invention relates to a single-chain antiparallel coiled coil protein wherein L1 and L2 have an amino acid composition comprising at least 50%, 70%, 90%, or comprising 100% amino acids selected from the group consisting of glycine, alanine, serine, threonine, proline or derivatives thereof. Since the latter amino acids correspond to more standard (more usually selected) linker residues, this embodiment is preferred over the previous.

Another preferred embodiment of the present invention relates to a single-chain antiparallel coiled coil protein wherein L1 and L2 have an amino acid composition comprising at least 50%, 70%, 90%, or comprising 100% glycine and/or serine amino acids. Since the latter amino acids correspond to the most standard (most frequently selected) linker residues, this embodiment is preferred over the previous.

Another preferred embodiment of the present invention relates to a single-chain antiparallel coiled coil protein wherein the number of amino acid residues of each of L1 and L2 amounts to less than half of the number of amino acid residues of the heptad repeat sequence preceding the respective L1 or L2. Respecting this rule considerably lowers the risk of unintended folding (e.g., as a parallel coiled coil), as explained supra.

Another preferred embodiment of the present invention relates to a single-chain antiparallel coiled coil protein wherein amino acid residues near the termini of L1 and/or L2 stabilize the alpha-helical ends of the coiled coil structure. Possibilities to select such amino acids are well documented in the literature and are generally known as 'helix-capping amino acids or helix-capping motifs'.

Another preferred embodiment of the present invention relates to a single-chain antiparallel coiled coil protein wherein amino acid residues near the termini of L1 and/or L2 promote formation of a local turn in the structure. Possibilities to select such amino acids include, for example, the selection of helix-breaking amino acids such as glycine and proline, or helix-initiating amino acids such as serine or aspartic acid. Certain helix-capping motifs may also be applied for the same purpose. Alternatively, helix-loop-helix motifs may be applied as documented in the literature or observed in the protein data bank (PDB).

Another preferred embodiment of the present invention relates to a single-chain antiparallel coiled coil protein wherein conventional heptad positions 'e' and 'g' are occupied by glutamines. Computer modeling of antiparallel coiled coil molecules of the present invention suggested that glutamine pairs at said positions may form quasi-ideal interactions (i.e., energetically favorable hydrogen bonds) between antiparallel helices, thereby augmenting the global stability of the fold.

Another preferred embodiment of the present invention relates to a single-chain antiparallel coiled coil protein wherein conventional heptad positions 'b', 'c' and 'f' are polar, solubility-promoting amino acids. Since these positions are the most solvent-exposed, the exclusive selection of polar, and preferably charged, amino acids at these positions may considerably enhance the solubility of said protein.

Another preferred embodiment of the present invention relates to a single-chain antiparallel coiled coil protein, which folds in aqueous solution having a pH between 1 and 13, or between 2 and 12, or between 3 and 11, or between 4 and 10, or between 5 and 9. The pH range wherein a protein remains folded is an important determinant of its applicability. For example, insensitivity (tolerance) to extreme pH conditions may render it suitable for therapeutic applications wherein the protein needs to pass through, or perform its function in, the gastrointestinal tract. Further, pH-insensitive proteins may be resistant to the acidic conditions of the lysosomal pathway following endocytosis. Therefore, proteins of the present invention are preferably stable in the pH range 5-9, more preferably 4-10, 3-11, 2-12, and most preferably 1-13.

Another preferred embodiment of the present invention relates to a single-chain antiparallel coiled coil protein, which folds in aqueous solution having a temperature between 0°C and 100°C, or between 0°C to 80°C, or between 0°C to 60°C. Thermal stability is an important determinant of global stability (including proteolytic stability and long-term stability or 'shelf life') and therefore also preservation of function. Proteins of the present invention are preferably stable at temperature ranges 0-60°C, more preferably 0-80°C, and most preferably 0-100°C.

Another preferred embodiment of the present invention relates to a single-chain antiparallel coiled coil protein, which folds in aqueous solution having an ionic strength between 0 and 1.0 molar. Physiological conditions require stable folding and preservation of function at ionic strengths (largely corresponding to salt concentrations) of about 150 millimolar. Proteins of the present invention are preferably stable (and functionally active) at broader ranges of ionic strength, most preferably in the range 0-1 molar.

Another preferred embodiment of the present invention relates to a single-chain antiparallel coiled coil protein, which is used as a scaffold. Protein molecules of the present invention are highly useful as scaffolds, as explained supra.

Another preferred embodiment of the present invention relates to a single-chain antiparallel coiled coil protein, as shown in figure 15.

The proteins of the present invention are amenable to a vast number of modifications, using knowledge from the art, including (multiple) amino acid substitutions, introduction of non-natural amino acids, attachment of particular chemical moieties, peptidic extensions, labeling, avidity enhancement through self-concatenation, concatenation into fusion proteins, etc., without compromising (changing, destroying) the coiled coil fold of the protein. A number of such modifications can be formulated here to illustrate the intrinsic potential of the protein to be subject to advanced engineering steps. Concretely, the present inventors contemplate the following engineered constructs, which all include the protein of the present invention with all of its specified characteristics:

- any protein of the present invention may be modified in amino acid sequence, thereby creating one or more derivatives thereof;
- any protein or derivative may be modified, e.g., to enhance its stability;
- any protein or derivative may be modified, e.g., to enhance its folding kinetics;
- any protein or derivative may be modified, e.g., to enhance the correctness of its folded state;
- any protein or derivative may be modified, e.g., to enhance its binding affinity to a target compound;
- any protein or derivative may be modified, e.g., to enhance its binding specificity for a target compound;
- any protein or derivative may be modified, e.g., to enhance its solubility;
- any protein or derivative may be covalently linked to any other protein or proteinaceous molecule, either via its N- and/or C-terminal ends or via one or more of its side chains;
- any protein or derivative may be covalently linked to other copies of the same protein or derivative, e.g., to increase avidity;
- any protein or derivative may be covalently linked to any protein or derivative with different binding properties, e.g., to provide bi- or multispecificity;
- any protein or derivative may be covalently linked to any existing natural or non-natural protein or protein domain or peptide that is not related to the present invention, including, without limitation, Fc domains, Fc receptor, serum albumin, fluorescent proteins, protein molecules of another type, etc.;
- any protein or derivative may be covalently linked to one or more detection tags;
- any protein or derivative may be covalently linked to one or more purification tags;
- any protein or derivative may be covalently linked to organic compounds by way of a chemical reaction with one or more protein side-chain moieties;
- any protein or derivative may be glycosylated;
- any protein or derivative may be PEGylated.

In view of the fact that the protein of the present invention, and derivatives thereof, form stable and compact structures, they may be constructed or manipulated, in principle, by all techniques applicable to proteins.

The protein molecules of the present invention can be made synthetically according to techniques well-known in the art or produced via genetic engineering using techniques that are also well-known in the art. When made with genetic engineering techniques, the protein molecules of the invention are encoded by polynucleotides (also referred to herein as nucleic acids), preferably DNA or RNA. The protein molecules of the invention can be encoded by any nucleic acid in accordance with the degeneracy of the genetic code of the host organism in which the protein molecule is made.

The polynucleotides (also referred to herein as nucleic acids) of the present invention can be incorporated into a recombinant vector, for example a cloning or expression vector. The term 'vector' includes expression vectors, transformation vectors and shuttle vectors. The term 'expression vector' means a construct capable of in vivo or in vitro expression. The term 'transformation vector' means a construct capable of being transferred from one entity to another entity - which may be of the same species or may be of a different species. If the construct is capable of being transferred from one species to another - such as from a viral vector such as MMLV or FIV to a human or mammalian primary cell or cell line, then the transformation vector is sometimes referred to as a "shuttle vector". A large variety of expression systems may be used in different hosts. For example, episomal, chromosomal and virus-derived systems (e.g. vectors derived from bacterial plasmids, bacteriophage, papova virus such as SV40, vaccinia virus, adenovirus, and retrovirus). The DNA sequence can be inserted into the vector by a variety of techniques. In general the DNA sequence is inserted into an appropriate restriction endonuclease site by procedures known in the art and deemed to be within the scope of those skilled in the art. The DNA sequence in the expression vector is linked operatively to appropriate control sequences that direct mRNA synthesis (i.e., the promoter). The vectors of the present invention may be transformed into a suitable host cell as described below to provide for expression of a protein molecule of the present invention. Thus, in a further aspect, the invention provides a process for preparing protein molecules according to the present invention which comprises cultivating a host cell transformed or transfected with an expression vector as described above under conditions to provide for expression by the vector of a coding sequence encoding the protein molecules, and recovering the expressed protein molecules. The vectors may be, for example, plasmid, virus or bacteriophage (phage) vectors provided with an origin of replication, optionally a promoter for the expression of the polynucleotide and optionally a regulator of the promoter. The vectors of the present invention may contain one or more selectable marker genes. The most suitable selection systems for industrial micro-organisms are those formed by the group of selection markers which do not require a mutation in the host organism. Examples of fungal selection markers are the genes for acetamidase (amdS), ATP synthetase, subunit 9 (oliC), orotidine-5'-phosphate-decarboxylase (pvrA), phleomycin and benomyl resistance (benA).

Examples of non-fungal selection markers are the bacterial G418 resistance gene (this may also be used in mammalian cells, yeast, but not in filamentous fungi), the ampicillin resistance gene (E. coli), the neomycin resistance gene (mammalian cells) and the E. coli uidA gene, coding for beta-glucuronidase (GUS). Vectors may be used in vitro, for example for the production of RNA or used to transfect or transform a host cell. Thus, polynucleotides or nucleic acids of the present invention can be incorporated into a recombinant vector (typically a replicable vector), for example a cloning or expression vector. The vector may be used to replicate the nucleic acid in a compatible host cell. Thus, in a further embodiment, the invention provides a method of making polynucleotides of the present invention by introducing a polynucleotide of the present invention into a replicable vector, introducing the vector into a compatible host cell, and growing the host cell under conditions which bring about replication of the vector. The vector may be recovered from the host cell. Suitable host cells are described below in connection with expression vectors. The term 'host cell' - in relation to the present invention - includes any cell that could comprise the nucleotide sequence coding for the recombinant protein according to the present invention and/or products obtained therefrom, wherein a promoter can allow expression of the nucleotide sequence according to the present invention when present in the host cell. Thus, a further embodiment of the present invention provides host cells transformed or transfected with a polynucleotide of the present invention.

Preferably said polynucleotide is carried in a vector for the replication and expression of said polynucleotide. The cells will be chosen to be compatible with the said vector and may, for example, be prokaryotic (for example, bacterial cells), or eukaryotic (i.e. mammalian, fungal, insect and yeast cells). Introduction of polynucleotides into host cells can be effected by methods as described in Sambrook, et al., eds. (1989) Molecular Cloning: A Laboratory Manual, Cold Spring Harbor Laboratory Press, New York, NY, USA. These methods include, but are not limited to, calcium phosphate transfection, DEAE-dextran-mediated transfection, cationic lipid-mediated transfection, electroporation, transvection, microinjection, transduction, scrape loading, and ballistic introduction. Examples of representative hosts include, bacterial cells (e.g., E. coli, Streptomyces); fungal cells such as yeast cells and Aspergillus; insect cells such as Drosophila S2 and Spodoptera SF9 cells; animal cells such as CHO, COS, HEK, HeLa, and 3T3 cells. The selection of the appropriate host is deemed to be within the scope of those skilled in the art. Depending on the nature of the polynucleotide encoding the protein molecule of the present invention, and/or the desirability for further processing of the expressed protein, eukaryotic hosts such as yeasts or other fungi may be preferred. In general, yeast cells are preferred over fungal cells because they are easier to manipulate. Examples of suitable expression hosts within the scope of the present invention are fungi such as Aspergillus species and Trichoderma species; bacteria such as Escherichia species, Streptomyces species and Pseudomonas species; and yeasts such as Kluyveromyces species and Saccharomyces species. By way of example, typical expression hosts may be selected from Aspergillus niger, Aspergillus niger var. tubigenis, Aspergillus niger var. awamori, Aspergillus aculeatis, Aspergillus nidulans, Aspergillus orvzae, Trichoderma reesei, Kluyveromyces lactis, Schizosaccharomyces pombe, Pichia pastoris and Saccharomyces cerevisiae. The use of suitable host cells - such as mammalian, yeast, insect and fungal host cells - may provide for post-translational modifications (e.g. myristoylation, glycosylation, truncation, and tyrosine, serine or threonine phosphorylation) as may be needed to confer optimal biological activity on recombinant expression products of the present invention. As indicated, the host cell can be a prokaryotic or a eukaryotic cell. An example of a suitable prokaryotic host is E. coli. Teachings on the transformation of prokaryotic hosts are well documented in the art, for example see Sambrook et al. (Molecular Cloning: A Laboratory Manual, 2nd edition, 1989, Cold Spring Harbor Laboratory Press, New York, NY, USA) and Ausubel et al. (Current Protocols in Molecular Biology (1995), John Wiley & Sons, Inc.). In a preferred embodiment, the transformed host is a mammalian cell or, for example, an insect cell, wherein introduction of polynucleotides into said host cells can be effected by methods as described in, for example, Sambrook et al. (Molecular Cloning: A Laboratory Manual, 2nd edition, 1989, Cold Spring Harbor Laboratory Press, New York, NY, USA). These methods include, but are not limited to, calcium phosphate transfection, DEAE-dextran-mediated transfection, cationic lipid-mediated transfection, electroporation, transvection, microinjection, transduction, scrape loading, and ballistic introduction. In another embodiment the transgenic organism can be a yeast. In this regard, yeast have also been widely used as a vehicle for heterologous gene expression. The species Saccharomyces cerevisiae has a long history of industrial use, including its use for heterologous gene expression. Expression of heterologous genes in Saccharomyces cerevisiae has been reviewed by Goodey et al. (1987, Yeast Biotechnology, D. R. Berry et al., eds, pp 401-429, Allen and Unwin, London) and by King et al. (1989, Molecular and Cell Biology of Yeasts, E. F. Walton and G. T. Yarronton, eds, pp 107-133, Blackie, Glasgow). According to the present invention, the production of the protein molecule of the present invention can be effected by the culturing of eukaryotic or prokaryotic expression hosts, which have been transformed with one or more polynucleotides of the present invention, in a conventional nutrient fermentation medium. The selection of the appropriate medium may be based on the choice of expression hosts and/or based on the regulatory requirements of the expression construct. Such media are well-known to those skilled in the art. The medium may, if desired, contain additional components favouring the transformed expression hosts over other potentially contaminating micro-organisms.

EXAMPLES

Example 1. Amino acid sequence of a synthetic peptide with core and non-core residues.

This example provides the amino acid sequence of a specific peptide which relates to the present invention. The amino acid sequence, AIAAIQKQIAAIQKQIAAIQKQIA, is presented in single-letter notation, wherein A refers to alanine, I to isoleucine, Q to glutamine, and K to lysine. The peptides with this amino acid sequence form triple-stranded, alpha-helical coiled coil complexes by way of their isoleucine and leucine amino acid residues forming a hydrophobic core (center, interior) and the other residues being oriented towards solvent. The artificial peptide comprises three heptad repeats labeled 'HR1', 'HR2' and 'HR3' in Figure 1.

The Figure 1 is a schematic representation of the amino acid sequence of an artificial peptide comprising heptad repeats (HRx), core residues (black boxes), non-core residues (gray boxes) and flanking regions (white boxes). The peptide further comprises a C-terminal heptad core residue labeled 't'. The peptide further comprises N- and C-terminal flanking fragments labeled 'N' and 'C', respectively. Each heptad repeat residue is further annotated with indices 'a' to 'g' and a number corresponding to the heptad repeat number. Core residues are located at a- and d-positions. All 6 core residues of the three full heptad repeats are isoleucines. The isoleucine residue labeled 'a4' belongs to the partial heptad repeat 't'. The heptad repeats HR1, HR2 and HR3 and the partial heptad repeat 't' together make up a heptad repeat sequence, starting with core residue a1 and ending with core residue a4.

Example 2. Principles of a triple-stranded, alpha-helical coiled coil complex.

Heptad core residues are shielded from solvent in triple-stranded, alpha-helical coiled coil complexes, as illustrated in Figure 2. Non-covalent interactions between contacting core residues (positions A and D in Figure 2) provide the main thermodynamic driving force for the peptides to adopt such fold.

The Figure 2 is a helical wheel representation of triple-stranded, alpha-helical coiled coil structures. The left panel shows a top view on a parallel coiled coil. The right panel shows a top view on an antiparallel coiled coil. The middle panel shows the linear sequence of heptad repeat positions. Only one heptad repeat is displayed for clarity reasons. Different shades are used to indicate specific topological positions.

The core residues (positions A and D) are fully buried in the complex and are not solvent accessible. The non-core residues (positions B, C, E, F and G) are at least partially solvent-accessible (positions E, G less than B, C, and positions B, C less than F) and are susceptible to amino acid substitutions without (major) implications for the stability of the complex.

Example 3. Alpha-helical structure and reversible folding/unfolding.

Peptidic alpha-helical coiled coils do not form the subject of the present invention because they do not fold into a single-chain protein. However, the single-chain proteins of the present invention do comprise a trimeric coiled coil region. Evidently, connecting the N- and C-terminal ends by linker fragments can (will) influence the folding kinetics, but the essential physical properties of the 'excised' coiled coil peptides are expected to be generally preserved. Hence, peptidic coiled coils may serve as a study system.

To demonstrate quantitative formation of alpha-helical secondary structure of a reference artificial peptide in solution, the inventors have synthesized the peptide with the amino acid sequence Ac-MSIEEIQKQQAAIQKQIAAIQKQIYRMTP-NH2 and recorded the circular dichroism (CD) spectrum. The amino acid sequence is given in single-letter code; Ac- and -NH2 mean that the peptide was acetyl-initiated and amide-terminated, respectively. This peptide is to be considered as a derivative of the reference peptide composed of the triple heptad repeat sequence (IAAIQKQ)3, with modifications at the amino- (N-) and carboxy- (C-) terminal ends to improve the alpha-helical nature of the termini (often referred to as capping). More specifically, the flanking residues Ac-MS- were attached at the N-terminus, in combination with the substitution of two consecutive glutamic acid residues (EE) for the two alanine residues (AA) in the first heptad of the reference sequence. Furthermore, the flanking residues - IYRMTP-NH2 were attached at the C-terminus, such that the amino acids isoleucine (I) and methionine (M) are located at conventional heptad a- and d-positions, allowing this flanking sequence to form an extra, though incomplete, heptad. The tyrosine (Y) was introduced at a solvent-oriented b-position to enable spectrophotometric concentration determination. The arginine (R), threonine (T) and proline (P-NH2) residues were introduced to improve C-terminal helical capping. In addition, the isoleucine (I) residue at the a-position of the second heptad was replaced by a glutamine (Q) residue to force the coiled coil-forming peptides to associate in the correct (intended) way, i.e., to ascertain formation of a trimeric complex and to avoid possible heptad register shifts [Eckert et al., J Mol Biol 1998, 284:859-865].

The said synthesized peptide was dissolved at a concentration of 292 microM in 20 mM phosphate buffer (PBS), 150 mM NaCl, pH 7.2. The CD spectra were measured between 200 and 250 nM, at 5°C and 90°C (Figure 3).

The spectrum at 5°C was indicative of a high alpha-helical secondary structure content, in agreement with the expectation that all heptad regions, but not all of the flanking residues, would assemble as alpha-helical coiled coils. The spectrum at 90 degrees Celsius showed that the alpha-helical structure was greatly, but not completely, lost at elevated temperatures.

In order to analyze whether the temperature-induced transition between helical and non-helical states was reversible, a forward (up) and backward (down) thermal scan was performed on the same sample, by recording the CD signal at 222 nM as a function of temperature at a scanning rate of about 1 degree Celsius per minute (Figure 4). It was observed that the up and down scans almost perfectly coincided, thereby confirming the quantitative unfolding and refolding of the peptides in the sample.

It was further analyzed whether the thermal unfolding curve of Figure 4 conformed to the thermodynamic equations describing the equilibrium folding/unfolding reaction between three molecules free (monomeric) peptide and one entity of folded (trimeric) complex. This reaction is generally written as 3 peptide <=> peptide$_3$ wherein '<=>' refers to a chemical equilibrium, 'peptide' to a monomeric peptide in solution and 'peptide$_3$' to a trimeric entity in the folded (assembled, associated) state. This thermal unfolding curve was fitted to the theoretic equations:

$$\theta(T) = \theta_M(T) + \left(\theta_T(T) - \theta_M(T)\right)\left(1 + \sqrt[3]{F\left(-\frac{1}{2} + \sqrt{\frac{1}{4} + \frac{F}{27}}\right)} + \sqrt[3]{F\left(-\frac{1}{2} - \sqrt{\frac{1}{4} + \frac{F}{27}}\right)}\right)$$

wherein

$$F = \frac{\exp\left(-\frac{\Delta H_t}{RT}(1 - T/T_t) - \frac{\Delta C_p}{RT}\left(T - T_t - T\ln(T/T_t)\right)\right)}{4}$$

and

$T$ = the temperature, in degrees Kelvin, of the sample
$\theta(T)$ = the CD-signal [theta]$_{222\ nm}$, in deg cm$^2$ dmol$^{-1}$, as a function of $T$
$\theta_M(T) \equiv$ the CD-signal for 100% free (monomeric) peptide as a function of $T$
$\theta_T(T) \equiv$ the CD-signal for 100% associated (trimeric) peptide as a function of $T$
$T_t \equiv$ the transition temperature, where 50% of the total peptide concentration is associated $\Delta H_t \equiv$ the enthalpy difference, in kJ per mole peptide, between mono- and trimeric, states $\Delta C_p \equiv$ the heat capacity difference, in J mol$^{-1}$ K$^{-1}$, between mono- and trimeric states R $\equiv$ the ideal (universal) gas constant $\equiv$ 8.31 J mol$^{-1}$ K$^{-1}$

The results of this fitting operation are shown in Figure 5. It was found that the theoretic curve almost perfectly coincided over the entire temperature range with the experimental curve, thereby confirming trimeric association of the peptides.

Figure 5 represents fitting of a theoretic equation for trimeric association to experimental data. The experimental data are taken from Figure 4, curve labeled 'UP'. The theoretic equations used are listed *supra*. The fitted parameters (fitting results) are listed at the right in Figure 5. 'Transit. T' corresponds to $T_t$, but is expressed in degrees Celsius. The parameter 'delta Cp' was kept constant at 3.0 kJ mol$^{-1}$ K$^{-1}$. The parameters 'theta$_M$($T$)' and 'theta$_T$($T$)' were treated as linear functions of $T$, resulting in the dotted straight lines described by the respective offsets and slopes indicated at the right in the figure. 'RMS Resid.' refers to the root-mean-square of the differences between experimental and theoretic data points. The fitted (theoretic) curve itself is plotted in white on the figure and coincides over the entire temperature range with the experimental data points shown in black.

Example 4. Usage of all-isoleucine core residues.

To analyze whether the glutamine residue at position a of the second heptad in the reference peptide of Example 3 was required for correct (intended) folding into a trimeric coiled coil, this residue was replaced by isoleucine, resulting in a peptide named 'Q2aI' having a sequence with isoleucine at all core positions (except methionine within the C-terminal flanking fragment). For this purpose, the peptide with the following sequence was synthesized: Ac-MSIEEIQKQIAAIQKQIAAIQKQIYRMTP-NH2.

Figure 6 shows the thermal denaturation curve for a sample preparation of the Q2aI peptide under the same conditions as in Example 3. The global CD signal was somewhat lower than expected, which could be due to an instrumental deviation, an error in the concentration determination, a lower purity, or a lower than expected alpha-helical content. Nevertheless, the main goal of this experiment was to examine the effect of the glutamine-to-isoleucine mutant on the stability of the complex. It was therefore interesting to find that this variant showed extremely high resistance against thermal denaturation, i.e., it was extremely thermostable. The estimated transition temperature was around 97 degrees Celsius, although the latter was difficult to determine because of incompleteness of the transition. Also, the down-scan showed full recovery of the CD signal, indicating full reversibility.

To confirm that the assembled complex had the correct molecular weight (MW), as expected for a trimer, the Q2aI peptide was submitted to analytical sedimentation equilibrium ultracentrifugation at 25000 rpm at a concentration of approximately 1 mg/ml. Figure 7 shows the linearized optical density (OD) curve in comparison with the theoretical curves for monomeric, dimeric and trimeric complexes. It was found that the experimental data points coincided very well with the trimeric model curve. From the slope of the linear regression line, the apparent molecular weight of 10500 Da was derived, in good agreement with the theoretic value of 10242 Da (3 times the MW of 3414 Da for a monomer).

To further confirm formation of trimeric complexes, the same Q2aI peptide was also analyzed by static light scattering. 200 microliter peptide at 1 mg/ml in PBS was put on a Superdex 75 10/300 GL gel filtration column connected to ultra-violet (UV), refractive index (RI) and static light scattering (SLS) detectors. Figure 8 shows the results. The signals (curves) from the three different detectors are labeled accordingly. A well-shaped light scattering peak was observed coinciding with a UV and RI peak. The apparent molecular weight derived for the UV peak was $12530 \pm 1510$ Da, again in good agreement with the expected value.

It was concluded that the use of all-isoleucine core residues had no adverse effect on the assembly of the peptides into trimers, as could be expected on the basis of theoretical considerations about potential (unintended) heptad register shifts. Instead, all tests indicated the proper and exclusive folding into trimers with the correct (expected) molecular weight. Furthermore, this all-isoleucine core peptide had a very high thermal stability, for it did not quantitatively unfold up to 95 degrees Celsius. Therefore, this peptide can be considered as a preferred trimeric coiled coil-forming peptide.

Example 5. Single-chain coiled coil scaffold constructs.

In order to examine whether single-chain coiled coil scaffolds could be derived from peptidic coiled coils by way of connecting termini of individual heptad repeat sequences (HRS) using structurally flexible linker fragments, three constructs with different linker lengths were designed, produced and tested. Concretely, the single-chain coiled coil scaffold molecules with the amino acid sequences listed in Figure 9 were constructed. These scaffolds were derived from the peptidic trimeric coiled coil scaffold of Example 4 (Q2aI). Gly/Ser-rich linkers of 8 and 16 amino acids in length were tested. These constructs are herein denoted as 'scQ2aI_L8' and 'scQ2aI_L16', respectively. In view of the definition of heptad repeat sequences (provided supra) starting and ending with a core residue, the N- and C-terminal capping residues methionine-serine ('MS') and threonine ('T'), respectively, are formally included in the linkers, and the sequences 'MGHHHHHHHHHHHHSSGHIEGRHMS' and 'TP' are considered as flanking sequences. The N-terminal flanking sequence (leader sequence) comprises a 10-His tag (HHHHHHHHHH) followed by a 'factor Xa' cleavage site (IEGRH).

The constructs were produced according to the following method. Genes coding for the constructs were retrieved. Nucleotide sequences were optimized to match the codon usage for expression in *E. coli.* The genes were provided in the pCR 4 TOPO plasmid and appended with a 3' -NdeI and a 5' -XhoI restriction site for subsequent sub-cloning in the pET16b vector (Novagen). The latter were transformed into the *E.coli* BL21(DE3)/pLysE strain and small-scale expression tests were performed. Briefly, 25 ml of medium containing the appropriate antibiotics (LB medium + 50 microg/ml ampicillin + 25 microg/ml chloramphenicol) was inoculated with an O/N culture (dilution 1/150x) and cells were grown at 37°C till OD600 reached about 0.65. Expression of the target proteins was then induced by the addition of 0.4 mM of IPTG and cells were further grown at either 37°C or 30°C. Culture aliquots were taken after 3.5 hours (t1, 37°C) and 5.5 hours (t2, 37°C and t2', 30°C) and analyzed on SDS-PAGE gels (10% acryl, Coomassie staining), together with a before-induction (to) sample. For all constructs, upon induction, a band appeared at about the expected MW.

To isolate protein from the soluble fraction, about 1.3 liter of culture was induced for 5.5 hours at 30°C. Cells were harvested, resuspended in a 50 mM Tris, 150 mM NaCl, pH 7.8 buffer and then disrupted by passing through a cell cracker. The soluble fraction was recovered by centrifugation and loaded onto a 5 ml column charged with Ni2+ for IMAC-based isolation of the target protein. The column was washed with 10 column volumes of buffer containing 20 mM of imidazole and a gradient of 20 to 600 mM of imidazole was used for the elution step. Protein containing fractions were pooled and concentrated from -15 to ~6 ml (Vivaspin MWCO 5 kDa, 2800 rpm). The proteins were further purified on a preparative gel filtration column (Superdex 75 16/90; 50 mM Tris, 150 mM NaCl, pH 7.8 as running buffer; two runs; ~3 ml loaded/run). The proteins eluted at around 130 ml; relevant fractions were pooled and concentrated to a final volume of ~10 ml (Vivaspin MWCO 5kDa, 2800 rpm). Calculated soluble expression levels were in the range 10 - 15 mg per liter bacterial culture.

Figure 10 shows the CD thermoscan for the scQ2aI_L16 construct in 20 mM PBS, 150 mM NaCl, pH 7.2. The thermoscan indicates that there is no thermal unfolding up to 90 degrees Celsius. This shows that the said construct is hyperthermostable, with a transition temperature exceeding 100 degrees Celcius.

To be able to observe a full transition, subsequent thermal unfolding experiments were performed in the presence of 6 M guanidinium hydrochloride (GuHCl). Figure 11 shows the thermal denaturation scans of scQ2al_L16 and scQ2al_L8 in 6 M GuHCl recorded by CD at 222 nm. The protein concentration was about 30 $\mu$M in the same PBS buffer. The scans were fitted to a two-state transition model and converted to fraction folded protein. The transition temperature of the scQ2al_L8 construct was found to be 7 degrees Celcius higher than that of the scQ2al_L16 construct. This result was not expected because only the L16 construct is supplied with linkers that are long enough to bridge the distance between the helical termini in parallel orientation ('overhand connection'). As described supra, for an overhand connection, the number of residues in the linker must be at least half the number of residues in the coiled coil helices'. Indeed, the 8-residue Gly/Ser-linker comprises less than 28/2 = 14 residues that are theoretically required, even if the capping residues are taken to be part of the linker (i.e., ignoring the fact that they need to allow reversal of chain direction, which also requires at least one or two residues). Thus, it was concluded that the higher thermostability of the scQ2al_L8 construct was in contradiction with a parallel coiled coil structure.

It was also considered that the too short linkers might induce local unfolding of one or more of the helical termini, and thereby still allow overhand closure in parallel orientation. This hypothesis was considered unlikely because such phenomenon would logically yield a less stable construct instead of the observed higher stability. Nevertheless, in order to exclude the latter possibility, a series of 'short' constructs was made comprising one less heptad in each alpha-helix. Concretely, the heptad repeat sequences of the new constructs consisted of the sequence IEEIQKQIAAIQKQIYRM (instead of IEEIQKQIAAIQKQIAAIQKQIYRM), with otherwise identical flanking segments and Gly/Ser linkers of the formula (GGSG)$_n$GG with n = 1, 2, 3, 4, yielding the respective constructs named 'short_L6', 'short_L10', 'short_L14' and 'short_L18'. It was reasoned that, if local unfolding would occur for the constructs with too short linkers (theoretically, for the L6 and L10 constructs), this should definitely lower their thermal stability. Therefore, these constructs were tested by CD-thermoscan at varying concentrations of GuHCl, and their transition temperatures were determined. Figure 12 shows the results. It was found that all four short constructs were less stable than the reference scQ2al_L16 by about 40 degrees Celcius at the same GuHCl concentrations, which was expected in view of the reduced coiled coil sizes. The relative stabilities of the four short constructs were highly similar under all conditions tested. At the highest GuHCl concentration (4 M), the construct with the shortest linker (short_L6) was again a little more stable than the others. It was therefore concluded that the hypothesis of local helical unwinding does not apply and, hence, that most likely all constructs are not parallel but, instead, antiparallel.

Example 6. NMR experiments.

To further provide evidence for the antiparallel fold of the reference coiled coil sequences of previous examples, $^{15}$N $^1$H HSQC NMR spectra were recorded for the constructs scQ2al_L16 and scQ2al_L8. Figure 13 shows the spectra, labeled 'L16' and 'L8', respectively. The side-chain and backbone amides roughly cluster in the upper-right and lower-left quadrant, respectively, and the more flexible linker backbone amides cluster in the upper-left quadrant. It is observed that the two spectra are highly similar, which is indicative of a type of fold that is independent of the linker length. Since the L8 linker is structurally incompatible with the parallel fold, it is concluded from these results that both are most likely antiparallel.

To provide additional evidence, a scQ2al_L16 derivative was made wherein a tryptophan (W) was introduced near the N-terminus of the second helix and a cysteine (C) near the C-terminus of the third helix. The full amino acid sequence was MGHHHHHHHHHHHHSSGHIEGRHMS-IEEIQKQIAAIQKQIAAIQKQIYRM-TGGSGGGSGGGSGGGSG-**_W_**S-IEEIQKQIAAIQKQIAAIQKQIYRM-TGGSGGGSGGGSGGGSGMS-IEEIQKQIAAIQKQIAAIQ**_C_**QIYRM-TP (mutations emphasized). If this sequence folds as a single-chain antiparallel coiled coil, then the two mutated positions should be proximal in space. The latter can be checked by way of conjugating the cysteine to a spin label and monitoring the effect of the spin label on the resonance of the tryptophan side-chain NHε. If the labeled cysteine and the tryptophan are in close proximity (i.e., preferably less than about 15 Å), then the NHε tryptophan signal should be significantly decreased. Treatment with vitamin C reduces the NO free radical and thereby restores (or increases) the NHε signal.

Figure 14 shows the NMR resonances of the said tryptophan NHε of the said mutated construct. The spin label used in the present experiment was 3-(2-iodoacetamido)-proxyl [i.e., 3-(2-iodoacetamido)-2,2,5,5 tetramethyl-1-pyrrolidinyloxy, free radical from Acros Organics cat. no. 224980250]. When comparing the signals for the untreated and vitamin C-treated samples (marked accordingly in Figure 14), it is observed that the signal of the untreated sample, bearing the free radical spin label, is indeed significantly decreased in comparison with the control sample with the reduced label. This proves that the tryptophan and cysteine are in close proximity, which, in view of the dimensions of the coiled coil structure (about 40 Å in length), is only possible in an antiparallel fold.

Example 7. Molecular modeling of parallel and antiparallel single-chain coiled coils

Figure 15 depicts 3-D molecular models of a parallel (left panel) and an antiparallel (right panel) triple-stranded single-chain coiled coil with the amino acid sequence of the construct scQ2aI_L16 (without N-terminal tag). The alpha-helices constituted of HRS1, HRS2 and HRS3 are respectively denoted as A, B and C. The two linker fragments are labeled L1 and L2, respectively.

The parallel model was constructed by homology modeling starting from the PDB structure 1GCM. The antiparallel model was constructed by reversing the orientation of the B helix in the parallel model, followed by shifting it along its helical axis until all side chains were free of atomic overlap. The latter was accomplished without modifying the rotameric structures of the core side chains. Linker fragments were modeled by a combination of interactive rotation around main-chain dihedral angles, molecular dynamics simulations and energy minimizations, while restraining the alpha-helical segments.

The models have been generated to examine whether antiparallel orientation is structurally feasible. Since all core-forming side chains could be placed in their most relaxed rotameric conformation, without leaving intermittent cavities, and resulting in credible packing of each heptad layer, it was concluded that antiparallel orientation is structurally possible, at least in the models shown.

TABLE 1

| Class | Fold | Superfamily | Protein | Species | PDB code |
|---|---|---|---|---|---|
| Coiled coil | Parallel | Triple coiled coil domain of C-type lectins | Mannose binding | Human | 1HUP |
| | | | | Rat | 1BUU, 1AFA, 1AFB, 1AFD, 1BCH, 1BCJ, 1FIF, 1FIH, 1KMB, 1KWT, 1KWU, 1KWV, 1KWW, 1KWX, 1KWY, 1KWZ, 1KXO, 7KX1, 1RTM, 2KMB, 3KMB, 4KMB |
| | | | Surfactant | Human | 1PWB, 1PW9, 1B08, 1M7L, 2GGU, 2GGX, 2ORJ, 2ORK, 2OS9 |
| | | | | Rat | 1R13, 1R14 |
| | | | Tetranectin | Human | 1HTN |
| | | Trimerization domain of TRAF | TRAF2 | Human | 1D01, 1CA4, 1CA9, 1CZY, 1CZZ, 1D00, 1D0A, 1D0J, 1F3V, 1QSC |
| | | | TRAF3 | Human | 1L0A, 1FLK, 1FLL. 1KZZ, 1RF3, 1ZMS, 2GKW |
| | | Leucine, zipper domain | GCN4 | Yeast | 1PIQ. 1GCM, 1ZIM, 1IJ3, 1IJ2, 1IJ1, 1IJ0, 1SWI, 1ZIJ, 1CE0, 1EBO, 1ENV, 1FAV, 2B9B, 1CZQ, 1GZL, 2Q3I, 2Q5U, 2Q7C, 2R3C, 2R5B, 2R5D, 2OXJ<br>Antiparallel; 1RB1 |
| | | Chicken cartilage matrix | | Chicken | 1AQ5 |
| | | Outer membrane lipoprotein | | E. coll | 1EQ7, 1KFM, 1KFN, 1JCC, 1JCD |

(continued)

| Class | Fold | Superfamily | Protein | Species | PDB code |
|---|---|---|---|---|---|
| | | Fibritin | | Bacteriophage T4 | 1AA0, 2BSG, 1AVY, 1OX3, 2IBL |
| | | MPN010-like | MPN010 | Mycoplasma pneumoniae | 2BA2 |
| | | Coronin 1 | | Mouse | 2AKF |
| | | DMPK | | Human | 1WT6 |
| | Stalk segment of viral fusion proteins | Influenza hemagglutinin | | Influenza A | 1QU1, 1EO8, 1HA0, 1HGD, 1HGE, 1HGF, 1HGG, 1HGH. 1HGI. 1HGJ, 1KEN, 1QFU, 2HMG, 2VIU, 3HMG, 4HMG, 5HMG, 1MQL, 1MQM, 1MQN, 1HTM, 1TI8, 1RD8, 1RUZ, 1RV0, 1RVT, 1RUY, 2FK0, 2IBX, 1RU7, 1RVX, 1RVZ |
| | | | | Influenza C | 1FLC |
| | | Virus ectodomain | Retrovirus gp41 | HIV type 1 | 1DF4, 1AIK, 1DF5, 1DLB, 1ENV, 1FAV, 1I5X, 1K33, 1K34, 1SZT, 2CMR, 1F23, 1QR9, 1I5Y, 1QR8, 1CZQ, 1GZL, 2Q3I, 2Q5U, 2Q7C, 2R3C, 2R5B, 2R5D |
| | | | | SIV | 1QBZ, 1QCE, 2EZO, 2EZP, 2EZQ, 2EZR, 2EZS, 1JPX, 2SIV, 1JQ0 |
| | | | | Visna | 1JEK |
| | | | HTLV-1 gp21 | HTLV type 1 | 1MG1 |
| | | | Ebo qp2 | Ebola virus | 2EBO, 1EBO |
| | | | MoMLV p15 | MoMLV | 1MOF |
| | | | Paramyxovlrus sv5 | SV5 strain w3 | 1SVF |
| | | | Paramyxovlrus hPIV3 | hPIV3 strain | 1ZTM |
| | | | Mumps virus | Mumps virus | 2FYZ |
| | | | NDV stalk | NDV | 1G5G |
| | | | HRSV fusion | HRSV | 1G2C |
| | | | HERV-FRD | Human | 1Y4M |
| | | | Nipah virus | Nipah virus | 1WP7 |
| | | | Hendra virus | Hendra virus | 1WP8 |
| | | Coronavirus S2 | E2 spike | MHV | 1WDG, 1WDF |
| | | | | SARS | 2BEQ, 1WNC, 1WYY, 1ZV8, 2BEZ, 1ZVB, 2FXP |
| | | | | NL63 | 2IEQ |

(continued)

| Class | Fold | Superfamily | Protein | Species | PDB code |
|---|---|---|---|---|---|
| Designed | Coiled serine | | | Synthetic | 2JGO<br>Antiparallel: 1COS |
| | Designed trimeric coiled coil | | VaLd | Synthetic | 1COI<br><br>Antiparallel: 1G6U |
| | | | Amyloidogenic design-1 | Synthetic | 1S9Z |
| | | | Unnamed design-1 | Synthetic | 1HQJ |
| | | | Unnamed design-2 | Synthetic | 1KYC |
| | Right-handed coiled coil | | Right-handed coiled coil trimer | Synthelic | 1TGG |
| All alpha proteins | Hypothetical protein Yhal | | | Bacillus Subtilis | 1SED |
| Membrane and cell surface | VP4 membrane Interaction domain | | | Rhesus rotavirus | 1SLQ |
| Small proteins | Resisting | | | Mouse | 1RGX,1RFX |
| | | | Resistin-like | Mouse | 1RH7 |
| | | G-protein binding domain | Rabaptin-5 | Human | Antiparallel: 1X79 |

## Claims

1. An isolated single-chain protein consisting of the formula HRS1-L1-HRS2-L2-HRS3, wherein HRS1, L1, HRS2, L2 and HRS3 represent amino acid sequence fragments that are covalently interconnected, said protein spontaneously folding in aqueous solution by way of the HRS1, HRS2 and HRS3 fragments forming a triple-stranded, antiparallel, alpha-helical coiled coil structure, and wherein

   each of HRS1, HRS2 and HRS3 is independently a heptad repeat sequence that is **characterized by** a n-times repeated 7-residue pattern of amino acid types, represented as (a-b-c-d-e-f-g-)n or (d-e-f-g-a-b-c-)n and a C-terminal partial heptad repeat ending with a heptad core residue located at an a- or d-position, wherein the pattern elements 'a' to 'g' denote conventional heptad positions at which said amino acid types are located and n is a number equal to or greater than 2, and

   at least 50%, 70%, 90%, or 100% of the conventional heptad positions 'a' and 'd' are occupied by amino acids selected from the group consisting of valine, isoleucine, leucine, methionine, phenylalanine, tyrosine, tryptophan, histidine, glutamine, threonine, serine, alanine or derivatives thereof, wherein at least 50%, 70%, 90%, or wherein 100% of the conventional heptad positions 'b', 'c', 'e', 'f and 'g' are occupied by amino acids selected from the group consisting of glycine, alanine, cysteine, serine, threonine, histidine, asparagine, aspartic acid, glutamine, glutamic acid, lysine, arginine or derivatives thereof, the resulting distribution of amino acid types enabling the identification of said heptad repeat sequences, and each of L1 and L2 is independently a linker consisting of 1 to 30 amino acid residues, this linker including any amino acid residue that cannot be unambiguously assigned to a heptad repeat sequence, and

   L1 and L2 have an amino acid composition comprising at least 50% amino acids selected from the group consisting of glycine, alanine, serine, threonine, proline or derivatives thereof.

2. The isolated protein of Claim 1, wherein at least 50%, 70%, 90%, or wherein 100% of the conventional heptad positions 'a' and 'd' are occupied by amino acids selected from the group consisting of valine, isoleucine, leucine, methionine or derivatives thereof.

3. The isolated protein of any of claims 1 to 2, wherein at least 50%, 70%, 90%, or wherein 100% of the conventional heptad positions 'a' and 'd' are occupied by isoleucines.

4. The isolated protein of any of claims 1 to 3, wherein L1 and L2 have an amino acid composition comprising at least 50%, 70%, 90%, or comprising 100% amino acids selected from the group consisting of glycine, alanine, cysteine, proline, serine, threonine, histidine, asparagine, aspartic acid, glutamine, glutamic acid, lysine, arginine or derivatives thereof.

5. The isolated protein of any of claims 1 to 4, wherein L1 and L2 have an amino acid composition comprising at least 70%, 90%, or comprising 100% amino acids selected from the group consisting of glycine, alanine, serine, threonine, proline or derivatives thereof.

6. The isolated protein of any of claims 1 to 5, wherein L1 and L2 have an amino acid composition comprising at least 50%, 70%, 90%, or comprising 100% glycine and/or serine amino acids.

7. The isolated protein of any of claims 1 to 6, wherein the number of amino acid residues of each of L1 and L2 amounts to less than half of the number of amino acid residues of the heptad repeat sequence preceding the respective L1 or L2.

8. The isolated protein of any of claims 1 to 7, wherein amino acid residues near the termini of L1 and/or L2 stabilize the alpha-helical ends of the coiled coil structure.

9. The isolated protein of any of claims 1 to 8, wherein amino acid residues near the termini of L1 and/or L2 promote formation of a local turn in the structure.

10. The isolated protein of any of claims 1 to 9, wherein conventional heptad positions 'e' and 'g' are occupied by glutamines.

11. The isolated protein of any of claims 1 to 10, wherein conventional heptad positions 'b', 'c' and 'f' are polar, solubility-promoting amino acids.

12. The isolated protein of any of claims 1 to 11, which folds in aqueous solution having a pH between 1 and 13, or between 2 and 12, or between 3 and 11, or between 4 and 10, or between 5 and 9.

13. The isolated protein of any of claims 1 to 12, which folds in aqueous solution having a temperature between 0°C and 100°C, or between 0°C to 80°C, or between 0°C to 60°C.

14. The isolated protein of any of claims 1 to 13, which folds in aqueous solution having an ionic strength between 0 and 1.0 molar.

15. Use of the isolated protein of any of claims 1 to 14 as a scaffold.

16. A nucleic acid encoding a protein according to any of claims 1 to 14.

17. A vector comprising a nucleic acid according to claim 16.

18. A host cell comprising a nucleic acid or vector according to claim 16 or 18.

19. A method for the production of a protein according to any of claims 1 to 14 comprising introducing a nucleic acid or vector into a host cell, culturing said host cell in a medium under conditions in which the nucleic acid is expressed and the protein is produced, and isolating the protein from said host cell and/or said medium.

**Patentansprüche**

1. Isoliertes einkettiges Protein, bestehend aus der Formel HRS1-L1-HRS2-L2-HRS3, wobei HRS1, L1, HRS2, L2 und HRS3 Aminosäuresequenzfragmente darstellen, die kovalent miteinander verbunden sind, wobei sich das Protein in wässriger Lösung durch Bildung einer dreisträngigen, antiparallelen, alpha-helikalen Coiled-Coil-Struktur durch die HRS1-, HRS2- und HRS3-Fragmente spontan faltet, und wobei
jedes von HRS1, HRS2 und HRS3 unabhängig eine Heptaden-Wiederholungssequenz ist, **gekennzeichnet durch** ein n-mal wiederholtes 7-Reste-Muster von Aminosäuretypen, dargestellt als (a-b-c-d-e-f-g-)n oder (d-e-f-g-a-b-c-)n, sowie eine C-terminale partielle Heptaden-Wiederholung, endend mit einem Heptaden-Kern-Rest, der sich an einer a- oder d-Position befindet, wobei die Musterelemente 'a' bis 'g' konventionelle Heptadenpositionen bezeichnen, an denen sich die Aminosäuretypen befinden, und n eine Zahl ist, die gleich 2 oder größer ist, und
mindestens 50 %, 70 %, 90 % oder 100 % der konventionellen Heptadenpositionen 'a' und 'd' **durch** Aminosäuren besetzt sind, die ausgewählt sind aus der Gruppe bestehend aus Valin, Isoleucin, Leucin, Methionin, Phenylalanin, Tyrosin, Tryptophan, Histidin, Glutamin, Threonin, Serin, Alanin oder Derivaten davon, wobei mindestens 50 %, 70 %, 90 %, oder wobei 100 % der konventionellen Heptadenpositionen 'b', 'c', 'e', 'f' und 'g' **durch** Aminosäuren besetzt sind, die ausgewählt sind aus der Gruppe bestehend aus Glycin, Alanin, Cystein, Serin, Threonin, Histidin, Asparagin, Asparaginsäure, Glutamin, Glutaminsäure, Lysin, Arginin oder Derivaten davon, wobei die resultierende Verteilung von Aminosäuretypen die Identifizierung der Heptaden-Wiederholungssequenzen ermöglicht, und
jedes von L1 und L2 unabhängig ein Linker ist, der aus 1 bis 30 Aminosäureresten besteht, wobei dieser Linker jeglichen Aminosäurerest einschließt, der nicht unzweideutig einer Heptaden-Wiederholungssequenz zugeordnet werden kann, und
L1 und L2 eine Aminosäurezusammensetzung besitzen, die mindestens 50 % Aminosäuren umfasst, die ausgewählt sind aus der Gruppe bestehend aus Glycin, Alanin, Serin, Threonin, Prolin oder Derivaten davon.

2. Isoliertes Protein gemäß Anspruch 1, wobei mindestens 50 %, 70 %, 90 %, oder wobei 100 % der konventionellen Heptadenpositionen 'a' und 'd' durch Aminosäuren besetzt sind, die ausgewählt sind aus der Gruppe bestehend aus Valin, Isoleucin, Leucin, Methionin oder Derivaten davon.

**3.** Isoliertes Protein gemäß mindestens einem der Ansprüche 1 bis 2, wobei mindestens 50 %, 70 %, 90 %, oder wobei 100 % der konventionellen Heptadenpositionen 'a' und 'd' durch Isoleucine besetzt sind.

**4.** Isoliertes Protein gemäß mindestens einem der Ansprüche 1 bis 3, wobei L1 und L2 eine Aminosäurezusammensetzung besitzen, umfassend mindestens 50 %, 70 %, 90 %, oder umfassend 100 % Aminosäuren, die ausgewählt sind aus der Gruppe bestehend aus Glycin, Alanin, Cystein, Prolin, Serin, Threonin, Histidin, Asparagin, Asparaginsäure, Glutamin, Glutaminsäure, Lysin, Arginin oder Derivaten davon.

**5.** Isoliertes Protein gemäß mindestens einem der Ansprüche 1 bis 4, wobei L1 und L2 eine Aminosäurezusammensetzung besitzen, umfassend mindestens 70 %, 90 %, oder umfassend 100 % Aminosäuren, die ausgewählt sind aus der Gruppe bestehend aus Glycin, Alanin, Serin, Threonin, Prolin oder Derivaten davon.

**6.** Isoliertes Protein gemäß mindestens einem der Ansprüche 1 bis 5, wobei L1 und L2 eine Aminosäurezusammensetzung besitzen, umfassend mindestens 50 %, 70 %, 90 %, oder umfassend 100 % Glycin- und/oder Serin-Aminosäuren.

**7.** Isoliertes Protein gemäß mindestens einem der Ansprüche 1 bis 6, wobei die Anzahl der Aminosäurereste von jedem von L1 und L2 sich auf weniger als die Hälfte der Anzahl der Aminosäurereste der Heptaden-Wiederholungssequenz beläuft, die dem jeweiligen L1 oder L2 vorausgeht.

**8.** Isoliertes Protein gemäß mindestens einem der Ansprüche 1 bis 7, wobei die Aminosäurereste nach den Enden von L1 und/oder L2 die alpha-helikalen Enden der Coiled Coil-Struktur stabilisieren.

**9.** Isoliertes Protein gemäß mindestens einem der Ansprüche 1 bis 8, wobei die Aminosäurereste nahe den Enden von L1 und/oder L2 die Bildung einer lokalen Windung in der Struktur begünstigen.

**10.** Isoliertes Protein gemäß mindestens einem der Ansprüche 1 bis 9, wobei die konventionellen Heptadenpositionen 'e' und 'g' durch Glutamine besetzt sind.

**11.** Isoliertes Protein gemäß mindestens einem der Ansprüche 1 bis 10, wobei die konventionellen Heptadenpositionen 'b', 'c' und 'f' polare, die Löslichkeit begünstigende Aminosäuren sind.

**12.** Isoliertes Protein gemäß mindestens einem der Ansprüche 1 bis 11, welches sich in wässriger Lösung mit einem pH zwischen 1 und 13 oder zwischen 2 und 12 oder zwischen 3 und 11 oder zwischen 4 und 10 oder zwischen 5 und 9 faltet.

**13.** Isoliertes Protein gemäß mindestens einem der Ansprüche 1 bis 12, welches sich in wässriger Lösung mit einer Temperatur zwischen 0°C und 100°C oder zwischen 0°C und 80°C oder zwischen 0°C und 60°C faltet.

**14.** Isoliertes Protein gemäß mindestens einem der Ansprüche 1 bis 13, welches sich in wässriger Lösung mit einer zwischen 0- und 1,0-molaren Ionenstärke faltet.

**15.** Verwendung des isolierten Proteins gemäß mindestens einem der Ansprüche 1 bis 14 als ein Gerüst.

**16.** Nukleinsäure, die ein Protein gemäß mindestens einem der Ansprüche 1 bis 14 kodiert.

**17.** Vektor, der eine Nukleinsäure gemäß Anspruch 16 umfasst.

**18.** Wirtszelle, die eine Nukleinsäure oder einen Vektor gemäß Anspruch 16 oder 18 umfasst.

**19.** Verfahren zur Herstellung eines Proteins gemäß mindestens einem der Ansprüche 1 bis 14, umfassend das Einbringen einer Nukleinsäure oder eines Vektors in eine Wirtszelle, das Kultivieren der Wirtszelle in einem Medium unter Bedingungen, bei welchen die Nukleinsäure exprimiert wird und das Protein hergestellt wird, und das Isolieren des Proteins aus der Wirtszelle und/oder dem Medium.

**Revendications**

**1.** Protéine isolée à chaîne unique constituée de la formule HRS1-L1-HRS2-L2-HRS3, où HRS1, L1, HRS2, L2 et HRS3 représentent des fragments de séquence d'acides aminés qui sont interconnectés de manière covalente, ladite protéine se repliant spontanément dans une solution aqueuse au moyen des fragments HRS1, HRS2 et HRS3 formant une structure de superhélice alpha-hélicoïdale, antiparallèle, triple brin, et où

chacun de HRS1, de HRS2 et de HRS3 est indépendamment une séquence de répétition d'heptade qui est **caractérisée par** un motif de 7 résidus répétés n fois de types d'acides aminés, représenté comme (a-b-c-d-e-f-g-)n ou (d-e-f-g-a-b-c-)n et une répétition d'heptade partielle C-terminale se terminant par un résidu de noyau d'heptade situé à une position a ou d, où les éléments de motif 'a' à 'g' désignent des positions d'heptade conventionnelles auxquelles lesdits types d'acides aminés sont situés et n est un nombre supérieur ou égal à 2, et

au moins 50%, 70%, 90%, ou 100% des positions d'heptade conventionnelles 'a' et 'd' sont occupées par des acides aminés choisis dans le groupe constitué de la valine, de l'isoleucine, de la leucine, de la méthionine, de la phénylalanine, de la tyrosine, du tryptophane, de l'histidine, de la glutamine, de la thréonine, de la sérine, de l'alanine ou de dérivés de ceux-ci, où au moins 50%, 70%, 90%, ou où 100% des positions d'heptade conventionnelles 'b', 'c', 'e', 'f' et 'g' sont occupées par des acides aminés choisis dans le groupe constitué de la glycine, de l'alanine, de la cystéine, de la sérine, de la thréonine, de l'histidine, de l'asparagine, de l'acide aspartique, de la glutamine, de l'acide glutamique, de la lysine, de l'arginine ou de dérivés de ceux-ci, la distribution résultante de types d'acides aminés permettant l'identification desdites séquences de répétition d'heptade, et

chacun de L1 et de L2 est indépendamment un lieur constitué de 1 à 30 résidus d'acides aminés, ce lieur comportant un résidu d'acide aminé quelconque qui ne peut pas être clairement attribué à une séquence de répétition d'heptade, et

L1 et L2 ont une composition d'acides aminés comprenant au moins 50% d'acides aminés choisis dans le groupe constitué de la glycine, de l'alanine, de la sérine, de la thréonine, de la proline ou de dérivés de celles-ci.

**2.** Protéine isolée de la revendication 1, dans laquelle au moins 50%, 70%, 90%, ou dans laquelle 100% des positions d'heptade conventionnelles 'a' et 'd' sont occupées par des acides aminés choisis dans le groupe constitué de la valine, de l'isoleucine, de la leucine, de la méthionine ou de dérivés de celles-ci.

**3.** Protéine isolée de l'une des revendications 1 à 2, dans laquelle au moins 50%, 70%, 90%, ou dans laquelle 100% des positions d'heptade conventionnelles 'a' et 'd' sont occupées par des isoleucines.

**4.** Protéine isolée de l'une des revendications 1 à 3, dans laquelle L1 et L2 ont une composition d'acides aminés comprenant au moins 50%, 70%, 90%, ou comprenant 100% d'acides aminés choisis dans le groupe constitué de la glycine, de l'alanine, de la cystéine, de la proline, de la sérine, de la thréonine, de l'histidine, de l'asparagine, de l'acide aspartique, de la glutamine, de l'acide glutamique, de la lysine, de l'arginine ou de dérivés de ceux-ci.

**5.** Protéine isolée de l'une des revendications 1 à 4, dans laquelle L1 et L2 ont une composition d'acides aminés comprenant au moins 70%, 90%, ou comprenant 100% d'acides aminés choisis dans le groupe constitué de la glycine, de l'alanine, de la sérine, de la thréonine, de la proline ou de dérivés de celles-ci.

**6.** Protéine isolée de l'une des revendications 1 à 5, dans laquelle L1 et L2 ont une composition d'acides aminés comprenant au moins 50%, 70%, 90%, ou comprenant 100% d'acide(s) aminé(s) glycine et/ou sérine.

**7.** Protéine isolée de l'une des revendications 1 à 6, dans laquelle le nombre de résidus d'acides aminés de chacun de L1 et de L2 représente moins de la moitié du nombre de résidus d'acides aminés de la séquence de répétition d'heptade précédant L1 ou L2 respectif.

**8.** Protéine isolée de l'une des revendications 1 à 7, dans laquelle des résidus d'acides aminés à proximité des extrémités terminales de L1 et/ou de L2 stabilisent les extrémités alpha-hélicoïdales de la structure de superhélice.

**9.** Protéine isolée de l'une des revendications 1 à 8, dans laquelle des résidus d'acides aminés à proximité des extrémités terminales de L1 et/ou de L2 favorisent la formation d'un tour local dans la structure.

**10.** Protéine isolée de l'une des revendications 1 à 9, dans laquelle les positions d'heptade conventionnelles 'e' et 'g' sont occupées par des glutamines.

**11.** Protéine isolée de l'une des revendications 1 à 10, dans laquelle les positions d'heptade conventionnelles 'b', 'c' et 'f' sont des acides aminés polaires, favorisant la solubilité.

12. Protéine isolée de l'une des revendications 1 à 11, qui se replie dans une solution aqueuse ayant un pH compris entre 1 et 13, ou entre 2 et 12, ou entre 3 et 11, ou entre 4 et 10, ou entre 5 et 9.

13. Protéine isolée de l'une des revendications 1 à 12, qui se replie dans une solution aqueuse ayant une température comprise entre 0°C et 100°C, ou entre 0°C et 80°C, ou entre 0°C et 60°C.

14. Protéine isolée de l'une des revendications 1 à 13, qui se replie dans une solution aqueuse ayant une force ionique comprise entre 0 et 1,0 molaire.

15. Utilisation de la protéine isolée de l'une des revendications 1 à 14 en tant qu'échafaudage.

16. Acide nucléique codant pour une protéine selon l'une des revendications 1 à 14.

17. Vecteur comprenant un acide nucléique selon la revendication 16.

18. Cellule hôte comprenant un acide nucléique ou un vecteur selon la revendication 16 ou 18.

19. Procédé pour la production d'une protéine selon l'une des revendications 1 à 14, comprenant l'introduction d'un acide nucléique ou d'un vecteur dans une cellule hôte, la mise en culture de ladite cellule hôte dans un milieu sous des conditions où l'acide nucléique est exprimé et la protéine est produite, et l'isolement de la protéine de ladite cellule hôte et/ou dudit milieu.

FIGURE 1

FIGURE 2

Parallel

Antiparallel

## FIGURE 3

## FIGURE 4

## FIGURE 5

| | |
|---|---|
| Transit. T (°C) = | 46.7 |
| ΔH, (kJ/mol) = | 177.9 |
| ΔC_p (kJ/mol/K) = | *3.0 |
| Offset 0 °C = | -34278 |
| Slope 0 °C = | 78.6 |
| Offset 100 °C = | -7718 |
| Slope 100 °C = | 47.1 |
| RMS Resid. = | 71.5 |

* parameter kept fixed

## FIGURE 6

## FIGURE 7

$y = 0.3477x - 0.0617$

$R^2 = 0.962$

◇ monomer

□ dimer

△ trimer

■ experimental

## FIGURE 8

FIGURE 9

| scQ2aI_L8 | a | d | a | d | a | d | a | d | |
|---|---|---|---|---|---|---|---|---|---|
| N | | | | | | | | | MGHHHHHHHHHHSSGHIEGRHMS |
| HRS1 | IEEIQKQIAAIQKQIAAIQKQIYRM | | | | | | | | |
| L1 | | | | | | | | | TGGSGGGSGMS |
| HRS2 | IEEIQKQIAAIQKQIAAIQKQIYRM | | | | | | | | |
| L2 | | | | | | | | | TGGSGGGSGMS |
| HRS3 | IEEIQKQIAAIQKQIAAIQKQIYRM | | | | | | | | |
| C | | | | | | | | | TP |
| Full | MGHHHHHHHHHHSSGHIEGRHMSIEEIQKQIAAIQKQIAAIQKQIYRMTGGSGGGSG MSIEEIQKQIAAIQKQIAAIQKQIYRMTGGSGGGSGMSIEEIQKQIAAIQKQIAAIQ KQIYRMTP | | | | | | | | |

| scQ2aI_L16 | a | d | a | d | a | d | a | d | |
|---|---|---|---|---|---|---|---|---|---|
| N | | | | | | | | | MGHHHHHHHHHHSSGHIEGRHMS |
| HRS1 | IEEIQKQIAAIQKQIAAIQKQIYRM | | | | | | | | |
| L1 | | | | | | | | | TGGSGGGSGGGSGGGSGMS |
| HRS2 | IEEIQKQIAAIQKQIAAIQKQIYRM | | | | | | | | |
| L2 | | | | | | | | | TGGSGGGSGGGSGGGSGMS |
| HRS3 | IEEIQKQIAAIQKQIAAIQKQIYRM | | | | | | | | |
| C | | | | | | | | | TP |
| Full | MGHHHHHHHHHHSSGHIEGRHMSIEEIQKQIAAIQKQIAAIQKQIYRMTGGSGGGSG GGSGGGSGMSIEEIQKQIAAIQKQIAAIQKQIYRMTGGSGGGSGGGSGGGSGMSIEE IQKQIAAIQKQIAAIQKQIYRMTP | | | | | | | | |

FIGURE 10

construct with a 16 residue linker

FIGURE 11

Thermal unfolding in 6 M GuHCl

FIGURE 12

FIGURE 13

FIGURE 14

FIGURE 15

Parallel

Antiparallel

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2008061886 W

### Non-patent literature cited in the description

- **SKERRA.** *J Mol Recognit,* 2000, vol. 13, 167-187
- **BINZ et al.** *Nat Biotechnol,* 2005, vol. 23, 1257-1268
- **HOSSE et al.** *Protein Sci,* 2006, vol. 15, 14-27
- **BURKHARD et al.** *Trends Cell Biol,* 2001, vol. 11, 82-88
- **WOLF et al.** *Protein Sci,* 1997, vol. 6, 1179-1189
- **LUPAS.** *Trends Biochem Sci,* 1996, vol. 21, 375-382
- **LUPAS et al.** *Science,* 1991, vol. 252, 1162-1164, http://www.russell.embl-heidelberg.de/cgi-bin/coils-svr.pl
- **SUZUKI et al.** *Protein Eng,* 1998, vol. 11, 1051-1055
- **HARBURY et al.** *Science,* 1993, vol. 262, 1401-1407
- *Nature,* 1994, vol. 371, 80-83
- **NAUTIYAL ; ALBER.** *Protein Sci,* 1999, vol. 8, 84-90
- **YU.** *Adv Drug Deliv Rev,* 2002, vol. 54, 1113-1129
- **LOVEJOY et al.** *Science,* 1993, vol. 259, 1288-1293
- **DRAGAN ; PRIVALOV.** *J mol Biol,* 2002, vol. 321, 891-908
- **DRAGAN et al.** *Biochemistry,* 2004, vol. 43, 14891-14900
- **HARRIS et al.** *J Mol Biol,* 1994, vol. 236, 1356-1368
- **HOLTON ; ALBER.** *Proc Natl Acad Sci USA,* 2004, vol. 101, 1537-1542
- **HARBURY et al.** *Nature,* 1994, vol. 371, 80-83
- **WALSHAW et al.** *J Struct Biol,* 2003, vol. 144, 349-361
- **ZHU et al.** *EMBO J,* 2004, vol. 23, 3909-3917
- Molecular Cloning: A Laboratory Manual. Cold Spring Harbor Laboratory Press, 1989
- **SAMBROOK et al.** Molecular Cloning: A Laboratory Manual. Cold Spring Harbor Laboratory Press, 1989
- **AUSUBEL et al.** Current Protocols in Molecular Biology. John Wiley & Sons, Inc, 1995
- **GOODEY et al.** Yeast Biotechnology. Allen and Unwin, 1987, 401-429
- **KING et al.** Molecular and Cell Biology of Yeasts. Blackie, 1989, 107-133
- **ECKERT et al.** *J Mol Biol,* 1998, vol. 284, 859-865